# EUROPEAN PATENT APPLICATION

(11) **EP 4 389 768 A1**
(43) Date of publication of application: **26.06.2024**
(21) Application number: 22857818.3
(22) Date of filing: 16.08.2022
(51) Int. Cl.: C07K 16/28, A61K 39/395, A61P 35/00

(54) **ANTI-B7-H4 ANTIBODY, AND PREPARATION METHOD THEREFOR AND USE THEREOF**

(30) Priority: 18.08.2021 CN 202110951949
(71) Applicant: Harbour Biomed (Shanghai) Co., Ltd, Shanghai 201203 (CN)
(72) Inventor: WU, Xiaodong, Shanghai 201203 (CN); WANG, Yongqiang, Shanghai 201203 (CN); JIA, Gezi, Shanghai 201203 (CN); ZHAO, Chuchu, Shanghai 201203 (CN); CHEN, Fei, Shanghai 201203 (CN); HE, Yun, Shanghai 201203 (CN); RONG, Yiping, Shanghai 201203 (CN); DING, Yi, Shanghai 201203 (CN); LI, He, Shanghai 201203 (CN); NIU, Lei, Shanghai 201203 (CN)
(74) Representative: Murgitroyd & Company
(86) International application number: PCT/CN2022/112864
(87) International publication number: WO 2023/020507

(57) **Abstract**

Provided is an anti-B7-H4 antibody containing a VH, wherein the VH contains the following CDRs or a mutation thereof: CDR1 as shown in the amino acid sequence of SEQ ID NO: 4 or 5, CDR2 as shown in the amino acid sequence of SEQ ID NO: 15, and CDR3 as shown in the amino acid sequence of SEQ ID NO: 24 or 25, and wherein the mutation is an insertion, deletion or substitution of 3, 2 or 1 amino acid(s) in the amino acid sequences of the CDRs. The antibody has the activity of binding to human B7-H4 and cynomolgus monkey B7-H4. The antibody still retains the activity of binding to human B7-H4 and cynomolgus monkey B7-H4 when prepared into a B7-H4×CD3 bispecific antibody, and has a strong killing effect on tumor cells. The antibody induces a very low expression of nonspecific cytokines such as IL-6 and IFN-y, and exhibits a strong in vivo anti-tumor activity.

## Description

### TECHNICAL FIELD

The present invention relates to the field of biomedicine, in particular to an anti-B7-H4 antibody, a preparation method therefor, and use thereof. The present invention also relates to a bispecific antibody comprising the anti-B7-H4 antibody.

### BACKGROUND

Breast cancer, ovarian cancer, and endometrioma are common malignant tumors in women, wherein breast cancer ranks first among cancers in women in incidence, and the data from the International Agency for Research on Cancer (IARC) in 2018 show that the incidence rate of the breast cancer in the cancers in women all over the world is 24.2%. For the treatment of these cancers, immunotherapy and targeted therapy are the current hot spots. For example, Her2-targeting Herceptin has a good therapeutic effect on Her2 positive breast cancer. For triple negative breast cancer (TNBC), few therapeutic approaches exist at present due to the lack of corresponding targets, mainly including chemotherapy. FDA approved the PD-L1 inhibitor atezolizumab in combination with albumin-bound paclitaxel for the treatment of metastatic triple negative breast cancer (TNBC) in March 2019, but PD-L1 is not highly expressed in triple negative breast cancer. The treatment of ovarian cancer and endometrioma mainly includes surgery and chemoradiotherapy, the adjuvant therapy. Besides these means, other available targets for recurrent or metastatic tumors mainly include estrogen receptors, HER2, VEGF, PD1/PD-L1, and the like, but the treatment effect is limited.

B7-H4 is a relatively new member of the B7 family. The expression of the protein in normal tissues is very limited, and the protein is only expressed at low level on partial ductal epithelial cells of an organic entity, such as breast ducts and lobules, oviduct epithelium, endometrial gland, and the like. In contrast, B7-H4 is abundantly expressed in a variety of tumor tissues, such as in breast cancer cells, particularly triple negative breast cancer cells, ovarian cancer cells, endometrioma cells, and the like. High expression of B7-H4 is present in approximately half of tumors. In terms of expression profile, B7-H4 can be considered as a tumor-associated antigen with high specificity. In another aspect, B7-H4 is a new immune checkpoint molecule, and *in vitro* experiments prove that B7-H4 inhibits the proliferation and activation of T cells and the production of cytokines by interacting with its unknown T cell surface receptors. The tumor cells inhibit the activation of T cells through high expression of B7-H4 molecules and inhibitory macrophages with high expression of B7-H4 molecules in a tumor microenvironment, thereby realizing immune evasion. The expression profile of B7-H4 on tumors does not overlap with that of PD-L1. Treatment with B7-H4-targeting antibodies is a promising approach to treat B7-H4 expression-positive tumors.

Monoclonal antibodies against B7-H4, or antibody-drug conjugates, or bispecific antibodies are currently being developed by several pharmaceutical companies. Those of Genentech, BMS, Jounce, Jiangsu Hanson, etc., are in preclinical development stage. At present, FivePrime and Nextcure have the fastest progress with their anti-B7-H4 monoclonal antibodies currently in clinical phase I, and the mechanism is primarily activating T cells through ADCC and blocking of immune checkpoints. What is constructed is an anti-tumor associated antigen and anti-CD3 bispecific antibody, which is called a T cell engager. The bispecific antibody has strong tumor-associated antigen dependent T cell activation and tumor killing effect and may have even stronger curative effect than common monoclonal antibodies.

### SUMMARY

The present invention aims to solve the technical problems, i.e., overcome the defects of anti-B7-H4 antibodies in the prior art, provides an anti-B7-H4 antibody, a preparation method therefor, and use thereof, and also provides a bispecific antibody comprising the anti-B7-H4 antibody.

To solve the above technical problems, a first aspect of the present invention provides an anti-B7-H4 antibody comprising a heavy chain variable region (VH); wherein
the VH comprises the following complementary determining regions (CDRs) or mutations thereof: a CDR1 having the amino acid sequence set forth in SEQ ID NO: 4 or 5, a CDR2 having the amino acid sequence set forth in SEQ ID NO: 15, and a CDR3 having the amino acid sequence set forth in SEQ ID NO: 24 or 25;
wherein the mutation is an insertion, a deletion, or a substitution of 3, 2, or 1 amino acid on the amino acid sequences of the CDRs.

In the present application, "amino acid mutation" in the context like "insertion, deletion or substitution of 3, 2 or 1 amino acid" refers to a mutation of an amino acid in the sequence of a variant as compared to the sequence of an original amino acid, including the insertion, deletion or substitution of amino acids on the basis of the original amino acid sequence. An exemplary explanation is that the mutations to the CDRs may comprise 3, 2 or 1 amino acid mutation, and that the same or different numbers of amino acid residues can be optionally selected for the mutations to these CDRs, e.g., 1 amino acid mutation to CDR1, and no amino acid mutations to CDR2 and CDR3.

In the present application, the mutation may include mutations currently known to those skilled in the art, e.g., mutations that may be made to an antibody during the production or application of the antibody, such as mutations made to sites that may be present, especially those subjected to post-transcriptional modifications (PTMs) in CDR regions, including aggregation of antibodies, asparagine deamidation (NG, NS, NH, etc.) sensitive sites, aspartate isomerization (DG, DP) sensitive sites, N-glycosylation (N-{ P}S/T) sensitive sites, oxidation sensitive sites, and relevant mutations, or relevant mutations by lowering isoelectric point.

Preferably, the mutation of the CDR2 is 2 or 1 amino acid substitution of G2P, S4G, and S5R/D/E/T on the amino acid sequence set forth in SEQ ID NO: 15; the CDR2 has the amino acid sequence preferably set forth in any one of SEQ ID NOs: 11-14 and SEQ ID NOs: 16-18.

The above G2P generally means that the amino acid G at position 2 of the amino acid sequence set forth in SEQ ID NO: 15 is mutated to P, and other amino acid substitutions such as S4G, S5R/D/E/T, etc., have a definition rule similar to the G2P, which should be understood by those skilled in the art.

Preferably, the anti-B7-H4 antibody comprises a heavy chain variable region (VH), wherein the VH comprises the following complementary determining regions (CDRs) or mutations thereof: a CDR1 having the amino acid sequence set forth in SEQ ID NO: 4 or 5, a CDR2 having the amino acid sequence set forth in SEQ ID NOs: 11-18, and a CDR3 having the amino acid sequence set forth in SEQ ID NO: 24 or 25.

In a certain embodiment, the CDR1, the CDR2, and the CDR3 comprised in the VH in the anti-B7-H4 antibody have amino acid sequences set forth in SEQ ID NOs: 4, 11, and 24, respectively.

In a certain embodiment, the CDR1, the CDR2, and the CDR3 comprised in the VH in the anti-B7-H4 antibody have amino acid sequences set forth in SEQ ID NOs: 5, 12, and 25, respectively.

In a certain embodiment, the CDR1, the CDR2, and the CDR3 comprised in the VH in the anti-B7-H4 antibody have amino acid sequences set forth in SEQ ID NOs: 4, 17, and 24, respectively.

In a certain embodiment, the CDR1, the CDR2, and the CDR3 comprised in the VH in the anti-B7-H4 antibody have amino acid sequences set forth in SEQ ID NOs: 5, 18, and 25, respectively.

In a certain embodiment, the CDR1, the CDR2, and the CDR3 comprised in the VH in the anti-B7-H4 antibody have amino acid sequences set forth in SEQ ID NOs: 4, 13, and 24, respectively.

In a certain embodiment, the CDR1, the CDR2, and the CDR3 comprised in the VH in the anti-B7-H4 antibody have amino acid sequences set forth in SEQ ID NOs: 4, 14, and 24, respectively.

In a certain embodiment, the CDR1, the CDR2, and the CDR3 comprised in the VH in the anti-B7-H4 antibody have amino acid sequences set forth in SEQ ID NOs: 4, 15, and 24, respectively.

In a certain embodiment, the CDR1, the CDR2, and the CDR3 comprised in the VH in the anti-B7-H4 antibody have amino acid sequences set forth in SEQ ID NOs: 4, 16, and 24, respectively.

Preferably, framework regions of the VH are framework regions of a human VH. In a certain embodiment, the framework regions of the human VH comprise a FWR1 having the amino acid sequence set forth in SEQ ID NO: 2, a FWR2 having the amino acid sequence set forth in any one of SEQ ID NOs: 7-9, a FWR3 having the amino acid sequence set forth in any one of SEQ ID NOs: 20-22, and a FWR4 having the amino acid sequence set forth in SEQ ID NO: 26 or 27. In a certain embodiment, the VH comprises the amino acid sequence set forth in any one of SEQ ID NOs: 36-45 or a mutation thereof, wherein the mutation is a deletion, a substitution, or an addition of one or more amino acid residues on the amino acid sequence of the VH, and the amino acid sequence with the mutation has at least 85% sequence identity to the amino acid sequence of the VH and maintains or improves the binding of the antibody to B7-H4, wherein the at least 85% sequence identity is preferably at least 90% sequence identity, more preferably at least 95% sequence identity, and most preferably at least 99% sequence identity.

In a certain preferred embodiment, the heavy chain variable region VH comprises the amino acid sequence set forth in SEQ ID NO: 36. In a certain preferred embodiment, the heavy chain variable region VH comprises the amino acid sequence set forth in SEQ ID NO: 37. In a certain preferred embodiment, the heavy chain variable region VH comprises the amino acid sequence set forth in SEQ ID NO: 44. In a certain preferred embodiment, the heavy chain variable region VH comprises the amino acid sequence set forth in SEQ ID NO: 45. In a certain preferred embodiment, the heavy chain variable region VH comprises the amino acid sequence set forth in SEQ ID NO: 38. In a certain preferred embodiment, the heavy chain variable region VH comprises the amino acid sequence set forth in SEQ ID NO: 39. In a certain preferred embodiment, the heavy chain variable region VH comprises the amino acid sequence set forth in SEQ ID NO: 40. In a certain preferred embodiment, the heavy chain variable region VH comprises the amino acid sequence set forth in SEQ ID NO: 41. In a certain preferred embodiment, the heavy chain variable region VH comprises the amino acid sequence set forth in SEQ ID NO: 42. In a certain preferred embodiment, the heavy chain variable region VH comprises the amino acid sequence set forth in SEQ ID NO: 43.

In the present application, the amino acid sequences of the CDRs are shown according to the Chothia scheme. However, it is well known to those skilled in the art that the CDRs of an antibody can be defined in the art using a variety of methods, such as the Kabat scheme based on sequence variability (see Kabat et al., Sequences of Proteins of Immunological Interest, Fifth Edition, National Institutes of Health (U.S.), Bethesda, Maryland (1991)) and the Chothia scheme based on the location of the structural loop regions (see J Mol Biol 273: 927-48, 1997). In the technical solution of the present invention, the Combined scheme comprising the Kabat scheme and the Chothia scheme can also be used to determine the amino acid residues in a variable domain sequence. The Combined scheme combines the Kabat scheme with the Chothia scheme to obtain a larger range. See Table 1-3 in the present application for details. It will be understood by those skilled in the art that unless otherwise specified, the terms "CDR" and "complementary determining region" of a given antibody or a region (e.g., variable region) thereof are construed as encompassing complementary determining regions as defined by any one of the above known schemes described herein. Although the scope claimed in the present invention is the sequences shown based on the Chothia scheme, the amino acid sequences corresponding to the other schemes for numbering CDRs shall also fall within the scope of the present invention.

Thus, when it comes to defining an antibody with a particular CDR sequence defined in the present invention, the scope of the antibody also encompasses antibodies whose variable region sequences comprise the particular CDR sequences but whose claimed CDR boundaries differ from the particular CDR boundaries defined in the present invention due to the application of different schemes (e.g., different assignment system rules or their combinations).

Preferably, the anti-B7-H4 antibody is a full-length antibody, a Fab, a Fab', a F(ab')₂, a Fv, preferably a scFv, a bispecific antibody, a multispecific antibody, a heavy-chain antibody, or a single-domain antibody, or any other antibodies retaining the ability of an antibody to specifically bind to an antigen (which may be part of the ability of the antibody to specifically bind to the antigen), or monoclonal or polyclonal antibodies prepared from the above antibodies.

Preferably, the anti-B7-H4 antibody is a heavy-chain antibody. In a certain embodiment, the heavy-chain antibody comprises the amino acid sequence set forth in any one of SEQ ID NOs: 48-57 or a mutation thereof. The mutation is a deletion, a substitution, or an addition of one or more amino acid residues on the amino acid sequence, and the amino acid sequence with the mutation has at least 85% sequence identity to the amino acid sequence and maintains or improves the binding of the antibody to B7-H4, wherein the at least 85% sequence identity is preferably at least 90% sequence identity, more preferably at least 95% sequence identity, and most preferably at least 99% sequence identity. In a certain preferred embodiment, the heavy-chain antibody comprises the amino acid sequence set forth in SEQ ID NO: 48. In a certain preferred embodiment, the heavy-chain antibody comprises the amino acid sequence set forth in SEQ ID NO: 49. In a certain preferred embodiment, the heavy-chain antibody comprises the amino acid sequence set forth in SEQ ID NO: 56. In a certain preferred embodiment, the heavy-chain antibody comprises the amino acid sequence set forth in SEQ ID NO: 57. In a certain preferred embodiment, the heavy-chain antibody comprises the amino acid sequence set forth in SEQ ID NO: 50. In a certain preferred embodiment, the heavy-chain antibody comprises the amino acid sequence set forth in SEQ ID NO: 51. In a certain preferred embodiment, the heavy-chain antibody comprises the amino acid sequence set forth in SEQ ID NO: 52. In a certain preferred embodiment, the heavy-chain antibody comprises the amino acid sequence set forth in SEQ ID NO: 53. In a certain preferred embodiment, the heavy-chain antibody comprises the amino acid sequence set forth in SEQ ID NO: 54. In a certain preferred embodiment, the heavy-chain antibody comprises the amino acid sequence set forth in SEQ ID NO: 55.

In the present application, a "Fab fragment" consists of one light chain and CH1 and the variable region of one heavy chain. The heavy chain of a Fab fragment cannot form a disulfide bond with another heavy chain molecule. An "Fc" region is two heavy chain fragments comprising CH2 and CH3 domains of an antibody. The two heavy chain fragments are held together by two or more disulfide bonds and the hydrophobic interaction of the CH3 domains. The "Fab' fragment" contains one light chain and contains the VH domain, the CH1 domain, and the region between the CH1 and CH2 domains, so that interchain disulfide bonds can be formed between the two heavy chains of two Fab' fragments to provide a F(ab')₂ fragment. The "F(ab')₂ fragment" contains two light chains and two heavy chains comprising part of the constant region between the CH1 and CH2 domains, so that interchain disulfide bonds are formed between the two heavy chains. Thus, a F(ab')₂ fragment consists of two Fab' fragments held together by disulfide bonds between the two heavy chains. The term "Fv" refers to an antibody fragment consisting of the VL and VH domains of a single arm of an antibody, but lacks the constant region.

In the present application, the scFv (single chain antibody fragment) may be a conventional single chain antibody in the art, which comprises a heavy chain variable region, a light chain variable region, and a short peptide linker of 15-20 amino acids. In the scFv, the VL and VH domains are paired to form a monovalent molecule via a linker that enables them to produce a single polypeptide chain (see, e.g., Bird et al., Science 242:423-426 (1988) and Huston et al., Proc. Natl. Acad. Sci. USA 85:5879-5883 (1988)). Such scFv molecules may have a general structure: NH₂-VL-linker-VH-COOH or NH₂-VH-linker-VL-COOH. An appropriate linker in the prior art consists of repeated G₄S amino acid sequences or a variant thereof. For example, linkers having the amino acid sequence (G₄S)₄ (i.e., SEQ ID NO: 88) or (G₄S)₃ may be used, but variants thereof may also be used.

In the present application, the term "multispecific antibody" is used in its broadest sense to encompass antibodies having multi-epitope specificity. These multispecific antibodies include, but are not limited to: an antibody comprising a heavy chain variable region (VH) and a light chain variable region (VL), the VH-VL unit having multi-epitope specificity; an antibody having two or more VL and VH regions, each of the VH-VL units binding to a different target or a different epitope on a same target; an antibody having two or more single variable regions, each of the single variable regions binding to a different target or a different epitope on a same target; full-length antibodies, antibody fragments, bispecific antibodies (diabodies), triabodies, antibody fragments linked together covalently or non-covalently, and the like.

In the present application, the monoclonal antibody or mAb or Ab refers to an antibody targeting a single antigenic epitope obtained from a single clonal cell strain, which is not limited to eukaryotic, prokaryotic, or phage clonal cell strains.

In the present application, the "single-domain antibody", also referred to as "nanobody", refers to a VHH structure cloned from a heavy-chain antibody. It is the smallest unit known to be able to bind to a target antigen.

In the present application, the "heavy-chain antibody", also referred to as HCAb, refers to an antibody comprising only one heavy chain variable region (VHH) and two conventional CH2 and CH3 domains.

To solve the above technical problems, a second aspect of the present invention provides a bispecific antibody comprising a protein functional region A and a protein functional region B, wherein the protein functional region A and the protein functional region B target different antigens, wherein the protein functional region B targets B7-H4, and the protein functional region A targets non-B7-H4; the bispecific antibody is selected from the following a) or b):
a) the protein functional domain B is selected from the anti-B7-H4 antibody according to the first aspect of the present invention; or the protein functional region B comprises a HCDR1 set forth in SEQ ID NO: 72, a HCDR2 set forth in SEQ ID NO. 74, a HCDR3 set forth in SEQ ID NO: 76, a LCDR1 set forth in SEQ ID NO: 78, a LCDR2 set forth in SEQ ID NO: 80, and a LCDR3 set forth in SEQ ID NO: 82.
   Preferably, the bispecific antibody has a structure comprising a polypeptide chain 1, a polypeptide chain 2, and a polypeptide chain 3, wherein the polypeptide chain 1 is shown as formula N'-VL_A-CL-C', the polypeptide chain 2 is shown as formula N'-VH_A-CH1-hinge region-CH2-CH3-C', and the polypeptide chain 3 is shown as formula N'-VH_B1-linker-VH_B2-hinge region (which may also be a linker peptide) -CH2-CH3-C' or formula N'-VH_B-hinge region (which may also be a linker peptide) -CH2-CH3-C', wherein the VL_A and the VH_A are a VL and a VH, respectively, of the protein functional region A, the VH_B1 and the VH_B2 are VHs of the protein functional region B, sequences of the VH_B1 and the VH_B2 can be the same or different, and the VH_B1 and the VH_B2 can be linked via a linker peptide. The VH_A and the VL_A are heavy chain and light chain variable regions, respectively, of the protein functional region A; the VH_B and the VL_B are heavy chain and light chain variable regions, respectively, of the protein functional region B; the CL is a domain of a light chain constant region; the CH1, the CH2, and the CH3 are first, second, and third domains, respectively, of a heavy chain constant region.
b) the protein functional region B comprises a HCDR1 set forth in SEQ ID NO: 72, a HCDR2 set forth in SEQ ID NO. 74, a HCDR3 set forth in SEQ ID NO: 76, a LCDR1 set forth in SEQ ID NO: 78, a LCDR2 set forth in SEQ ID NO: 80, and a LCDR3 set forth in SEQ ID NO: 82;

Preferably, the bispecific antibody has a structure comprising a polypeptide chain 1, a polypeptide chain 2, and a polypeptide chain 3, wherein the polypeptide chain 1 is shown as formula N'-VL_A-CL-C', the polypeptide chain 2 is shown as formula N'-VH_A-CH1-hinge region-CH2-CH3-C', and the polypeptide chain 3 is shown as formula N'-VL_B-linker-VH_B-hinge region-CH2-CH3-C', wherein the VL_A and the VH_A are a VL and a VH, respectively, of the protein functional region A, and the VL_B and the VH_B are a VL and a VH of the protein functional region B.

In some preferred embodiments, the protein functional region A is an anti-CD3 antibody comprising a heavy chain variable region (VH) and a light chain variable region (VL), wherein the VH comprises a VH CDR1 having the amino acid sequence set forth in SEQ ID NO: 3, a VH CDR2 having the amino acid sequence set forth in SEQ ID NO: 10, and a VH CDR3 having the amino acid sequence set forth in SEQ ID NO: 23, and the VL comprises a VL CDR1 having the amino acid sequence set forth in SEQ ID NO: 29, a VL CDR2 having the amino acid sequence set forth in SEQ ID NO: 31, and a VL CDR3 having the amino acid sequence set forth in SEQ ID NO: 33. In a certain embodiment, the anti-CD3 antibody comprises a VH having the amino acid sequence set forth in SEQ ID NO: 35 and a VL having the amino acid sequence set forth in SEQ ID NO: 46. In a certain embodiment, the anti-CD3 antibody comprises a heavy chain having the amino acid sequence set forth in SEQ ID NO: 47 and a light chain having the amino acid sequence set forth in SEQ ID NO: 58.

In a certain preferred embodiment, the protein functional region A comprises a light chain variable region and a heavy chain variable region, wherein the light chain variable region VL comprises a LCDR1, a LCDR2, and a LCDR3 having amino acid sequences set forth in SEQ ID NOs: 29, 31, and 33, respectively, and the heavy chain variable region VH comprises a HCDR1, a HCDR2, and a HCDR3 having amino acid sequences set forth in SEQ ID NOs: 3, 10 and 23, respectively. The protein functional region B comprises a heavy chain variable region, wherein the heavy chain variable region VH comprises a HCDR1, a HCDR2, and a HCDR3 having amino acid sequences set forth in SEQ ID NOs: 4, 17, and 24, respectively. The amino acid sequences of the listed CDRs are shown according to the Chothia scheme. In a certain preferred embodiment, the protein functional region A comprises a light chain variable region and a heavy chain variable region, wherein the light chain variable region VL comprises a LCDR1, a LCDR2, and a LCDR3 having amino acid sequences set forth in SEQ ID NOs: 29, 31, and 33, respectively, and the heavy chain variable region VH comprises a HCDR1, a HCDR2, and a HCDR3 having amino acid sequences set forth in SEQ ID NOs: 3, 10 and 23, respectively. The protein functional region B comprises a heavy chain variable region, wherein the heavy chain variable region VH comprises a HCDR1, a HCDR2, and a HCDR3 having amino acid sequences set forth in SEQ ID NOs: 5, 18, and 25, respectively. The amino acid sequences of the listed CDRs are shown according to the Chothia scheme. In a certain preferred embodiment, the protein functional region A comprises a light chain variable region and a heavy chain variable region, wherein the light chain variable region VL comprises a LCDR1, a LCDR2, and a LCDR3 having amino acid sequences set forth in SEQ ID NOs: 29, 31, and 33, respectively, and the heavy chain variable region VH comprises a HCDR1, a HCDR2, and a HCDR3 having amino acid sequences set forth in SEQ ID NOs: 3, 10 and 23, respectively. The protein functional region B comprises a first heavy chain variable region and a second heavy chain variable region, wherein the first heavy chain variable region VH comprises a HCDR1, a HCDR2, and a HCDR3 having amino acid sequences set forth in SEQ ID NOs: 4, 17, and 24, respectively, and the second heavy chain variable region VH comprises a HCDR1, a HCDR2, and a HCDR3 having amino acid sequences set forth in SEQ ID NOs: 5, 18, and 25, respectively. The amino acid sequences of the listed CDRs are shown according to the Chothia scheme. In a certain preferred embodiment, the protein functional region A comprises a light chain variable region and a heavy chain variable region, wherein the light chain variable region VL comprises a LCDR1, a LCDR2, and a LCDR3 having amino acid sequences set forth in SEQ ID NOs: 29, 31, and 33, respectively, and the heavy chain variable region VH comprises a HCDR1, a HCDR2, and a HCDR3 having amino acid sequences set forth in SEQ ID NOs: 3, 10 and 23, respectively. The protein functional region B comprises a heavy chain variable region and a light chain variable region, wherein the heavy chain variable region VH comprises a HCDR1, a HCDR2, and a HCDR3 having amino acid sequences set forth in SEQ ID NOs: 72, 74, and 76, respectively, and the light chain variable region VL comprises a LCDR1, a LCDR2, and a LCDR3 having amino acid sequences set forth in SEQ ID NOs: 78, 80, and 82, respectively. The amino acid sequences of the listed CDRs are shown according to the Chothia scheme.

In a certain preferred embodiment, the protein functional region A comprises a light chain variable region and a heavy chain variable region, wherein the light chain variable region VL comprises the amino acid sequence set forth in SEQ ID NO: 46, and the heavy chain variable region VH comprises the amino acid sequence set forth in SEQ ID NO: 35. The protein functional region B comprises a heavy chain variable region, wherein the heavy chain variable region VH comprises the amino acid sequence set forth in SEQ ID NO: 44. In a certain preferred embodiment, the protein functional region A comprises a light chain variable region and a heavy chain variable region, wherein the light chain variable region VL comprises the amino acid sequence set forth in SEQ ID NO: 46, and the heavy chain variable region VH comprises the amino acid sequence set forth in SEQ ID NO: 35. The protein functional region B comprises a heavy chain variable region, wherein the heavy chain variable region VH comprises the amino acid sequence set forth in SEQ ID NO: 45. In a certain preferred embodiment, the protein functional region A comprises a light chain variable region and a heavy chain variable region, wherein the light chain variable region VL comprises the amino acid sequence set forth in SEQ ID NO: 46, and the heavy chain variable region VH comprises the amino acid sequence set forth in SEQ ID NO: 35. The protein functional region B comprises a first heavy chain variable region and a second heavy chain variable region, wherein the first heavy chain variable region VH comprises the amino acid sequence set forth in SEQ ID NO: 44, and the second heavy chain variable region VH comprises the amino acid sequence set forth in SEQ ID NO: 45. In a certain preferred embodiment, the protein functional region A comprises a light chain variable region and a heavy chain variable region, wherein the light chain variable region VL comprises the amino acid sequence set forth in SEQ ID NO: 46, and the heavy chain variable region VH comprises the amino acid sequence set forth in SEQ ID NO: 35. The protein functional region B comprises a heavy chain variable region and a light chain variable region, wherein the heavy chain variable region VH comprises the amino acid sequence set forth in SEQ ID NO: 84, and the second heavy chain variable region VH comprises the amino acid sequence set forth in SEQ ID NO: 85.

In a certain preferred embodiment, the bispecific antibody comprises a polypeptide chain 1 having the amino acid sequence set forth in SEQ ID NO: 58, a polypeptide chain 2 having the amino acid sequence set forth in SEQ ID NO: 59, and a polypeptide chain 3 having the amino acid sequence set forth in SEQ ID NO: 60.

In a certain preferred embodiment, the bispecific antibody comprises a polypeptide chain 1 having the amino acid sequence set forth in SEQ ID NO: 58, a polypeptide chain 2 having the amino acid sequence set forth in SEQ ID NO: 59, and a polypeptide chain 3 having the amino acid sequence set forth in SEQ ID NO: 61.

In a certain preferred embodiment, the bispecific antibody comprises a polypeptide chain 1 having the amino acid sequence set forth in SEQ ID NO: 58, a polypeptide chain 2 having the amino acid sequence set forth in SEQ ID NO: 59, and a polypeptide chain 3 having the amino acid sequence set forth in SEQ ID NO: 62.

In a certain preferred embodiment, the bispecific antibody comprises a polypeptide chain 1 having the amino acid sequence set forth in SEQ ID NO: 58, a polypeptide chain 2 having the amino acid sequence set forth in SEQ ID NO: 59, and a polypeptide chain 3 having the amino acid sequence set forth in SEQ ID NO: 63.

In a certain preferred embodiment, the bispecific antibody comprises a polypeptide chain 1 having the amino acid sequence set forth in SEQ ID NO: 58, a polypeptide chain 2 having the amino acid sequence set forth in SEQ ID NO: 59, and a polypeptide chain 3 having the amino acid sequence set forth in SEQ ID NO: 64.

In a certain preferred embodiment, the bispecific antibody comprises a polypeptide chain 1 having the amino acid sequence set forth in SEQ ID NO: 58, a polypeptide chain 2 having the amino acid sequence set forth in SEQ ID NO: 59, and a polypeptide chain 3 having the amino acid sequence set forth in SEQ ID NO: 65.

In a certain preferred embodiment, the bispecific antibody comprises a polypeptide chain 1 having the amino acid sequence set forth in SEQ ID NO: 58, a polypeptide chain 2 having the amino acid sequence set forth in SEQ ID NO: 59, and a polypeptide chain 3 having the amino acid sequence set forth in SEQ ID NO: 69.

In a certain preferred embodiment, the bispecific antibody comprises a polypeptide chain 1 having the amino acid sequence set forth in SEQ ID NO: 58, a polypeptide chain 2 having the amino acid sequence set forth in SEQ ID NO: 59, and a polypeptide chain 3 having the amino acid sequence set forth in SEQ ID NO: 70.

In a certain preferred embodiment, the bispecific antibody comprises a polypeptide chain 1 having the amino acid sequence set forth in SEQ ID NO: 58, a polypeptide chain 2 having the amino acid sequence set forth in SEQ ID NO: 59, and a polypeptide chain 3 having the amino acid sequence set forth in SEQ ID NO: 86.

To solve the above technical problems, a third aspect of the present invention provides an isolated nucleic acid encoding the anti-B7-H4 antibody according to the first aspect of the present invention or the bispecific antibody according to the second aspect of the present invention.

The preparation method for the nucleic acid is a conventional preparation method in the art and preferably comprises the following steps: obtaining a nucleic acid molecule encoding the above antibody by gene cloning technology, or obtaining a nucleic acid molecule encoding the above antibody by artificial complete sequence synthesis.

It is known to those skilled in the art that substitutions, deletions, alterations, insertions, or additions may be appropriately introduced into the base sequence encoding the amino acid sequence of the above antibody to provide a polynucleotide homolog. The polynucleotide homolog of the present invention may be produced by substituting, deleting, or adding one or more bases of genes encoding the antibody sequence within a range in which the activity of the antibody is maintained.

To solve the above technical problems, a fourth aspect of the present invention provides a recombinant expression vector comprising the isolated nucleic acid according to the third aspect of the present invention.

The recombinant expression vector may be obtained by using conventional methods in the art, i.e., by linking the nucleic acid molecule of the present application to various expression vectors. The expression vector is any conventional vector in the art, provided that it can carry the aforementioned nucleic acid molecule.

Preferably, the expression vector comprises a eukaryotic cell expression vector and/or a prokaryotic cell expression vector.

To solve the above technical problems, a fifth aspect of the present invention provides a transformant comprising the recombinant expression vector according to the fourth aspect of the present invention.

The transformant may be prepared by using conventional methods in the art, e.g., by transforming the above recombinant expression vector into a host cell. The host cell of the transformant is any conventional host cell in the art, provided that it can enable the stable self-replication of the above recombinant expression vector and the effective expression of the nucleic acid carried in the vector. Preferably, the host cell is a prokaryotic and/or a eukaryotic cell, wherein the prokaryotic cell is preferably an *E*. *coli* cell such as TG1 or BL21 (expressing a single-chain antibody or Fab antibody), and the eukaryotic cell is preferably a HEK293 cell or a CHO cell (expressing a full-length IgG antibody). The preferred recombinant expression transformant of the present invention can be obtained by transforming the above recombinant expression plasmid into a host cell. The transformation method is a conventional transformation method in the art, preferably a chemical transformation method, a heat shock method, or electroporation.

To solve the above technical problems, a sixth aspect of the present invention provides a chimeric antigen receptor comprising the anti-B7-H4 antibody according to the first aspect of the present invention or the bispecific antibody according to the second aspect of the present invention.

To solve the above technical problems, a seventh aspect of the present invention provides a genetically modified cell comprising the chimeric antigen receptor according to the sixth aspect of the present invention.

Preferably, the genetically modified cell is a eukaryotic cell, preferably an isolated human cell, and more preferably an immune cell such as a T cell or an NK cell.

To solve the above technical problems, an eighth aspect of the present invention provides a method for preparing an anti-B7-H4 antibody or a bispecific antibody, which comprises the following steps: culturing the transformant according to the fifth aspect and obtaining the anti-B7-H4 antibody or the bispecific antibody from the culture.

To solve the above technical problems, a ninth aspect of the present invention provides an antibody-drug conjugate comprising an antibody moiety and a conjugate moiety, wherein the antibody moiety comprises the anti-B7-H4 antibody according to the first aspect of the present invention or the bispecific antibody according to the second aspect of the present invention, the conjugate moiety comprises a detectable label, a drug, a toxin, a cytokine, a radionuclide, an enzyme, or a combination thereof, and the antibody moiety and the conjugate moiety are conjugated via a chemical bond or a linker.

To solve the above technical problems, a tenth aspect of the present invention provides a pharmaceutical composition comprising the anti-B7-H4 antibody according to the first aspect of the present invention, the bispecific antibody according to the second aspect of the present invention, the chimeric antigen receptor according to the sixth aspect of the present invention, the genetically modified cell according to the seventh aspect of the present invention, and/or the antibody-drug conjugate according to the ninth aspect of the present invention.

Preferably, the pharmaceutical composition further comprises a pharmaceutically acceptable carrier.

More preferably, the pharmaceutical composition comprises 0.01%-99.99% of the above protein and/or the above antibody-drug conjugate and 0.01%-99.99% of a carrier, the percentage being the mass percentage of the pharmaceutical composition.

The route of administration for the pharmaceutical composition described herein is preferably parenteral administration, inj ection administration, or oral administration. The injection administration preferably includes intravenous injection, intramuscular injection, intraperitoneal injection, intradermal injection, subcutaneous injection, or the like. The pharmaceutical composition is in any conventional dosage form in the art, preferably in a liquid dosage form, a gas dosage form, a solid dosage form, or a semi-solid dosage form, i.e., it may be an aqueous solution, a non-aqueous solution, or a suspension, more preferably a tablet, a capsule, a granule, an injection, an infusion, or the like. More preferably, it is administered intravascularly, subcutaneously, intraperitoneally, or intramuscularly. Preferably, the pharmaceutical composition may also be administered as an aerosol or a spray, i.e., administered nasally; or it is administered intrathecally, intramedullarily, or intraventricularly. More preferably, the pharmaceutical composition may also be administered orally, by injection, nasally, transdermally, or transmucosally. The pharmaceutical composition of the present invention may be formulated into various dosage forms as required, and can be administered by a physician in the light of the patient's type, age, weight, and general disease state, route of administration, etc. The administration may be performed, for example, by injection or other therapeutic modalities.

The dose level at which the pharmaceutical composition of the present invention is administered can be adjusted depending on the amount of the composition to achieve the desired diagnostic or therapeutic outcome. The administration regimen may also be a single injection or multiple injections, or an adjusted one. The selected dose level and regimen are appropriately adjusted depending on a variety of factors including the activity and stability (i.e., half-life) of the pharmaceutical composition, the formulation, the route of administration, combination with other drugs or treatments, the disease or disorder to be detected and/or treated, and the health condition and previous medical history of the subject to be treated.

Preferably, the pharmaceutical composition may further comprise a combination therapeutic agent to perform a combination therapy, and the combination therapeutic agent comprises a chemotherapeutic agent, a radiotherapeutic agent, an immunosuppressant, and/or a cytotoxic drug. For combination therapy, the above antibody, the above antibody-drug conjugate, and/or other therapeutic or diagnostic agents may each be used as a single agent for use within any time frame suitable for performing the intended treatment or diagnosis. Thus, these single agents may be administered substantially simultaneously (i.e., as a single formulation or within several minutes or hours) or sequentially.

For additional guidance regarding formulations, doses, administration regimens, and measurable therapeutic outcomes, see Berkow et al., (2000) The Merck Manual of Medical Information and Merck & Co. Inc., Whitehouse Station, New Jersey; Ebadi (1998) CRC Desk Reference of Clinical Pharmacology, etc.

To solve the above technical problems, an eleventh aspect of the present invention provides use of the anti-B7-H4 antibody according to the first aspect of the present invention, the bispecific antibody according to the second aspect of the present invention, the chimeric antigen receptor according to the sixth aspect of the present invention, the genetically modified cell according to the seventh aspect of the present invention, the antibody-drug conjugate according to the ninth aspect of the present invention, and/or the pharmaceutical composition according to the tenth aspect of the present invention in the preparation of a medicament, a kit, and/or an administration device for treating and/or preventing cancer, or provides use of the anti-B7-H4 antibody according to the first aspect of the present invention, the bispecific antibody according to the second aspect of the present invention, the chimeric antigen receptor according to the sixth aspect of the present invention, the genetically modified cell according to the seventh aspect of the present invention, the antibody-drug conjugate according to the ninth aspect of the present invention, and/or the pharmaceutical composition according to the tenth aspect of the present invention in treating and/or preventing cancer.

Preferably, the cancer is selected from the group consisting of lung cancer, breast cancer such as triple negative breast cancer, ovarian cancer, endometrioma, stomach cancer, small intestine cancer, colon cancer, rectal cancer, pancreatic cancer, kidney cancer, bladder cancer, prostate cancer, bile duct cancer, esophageal cancer, osteosarcoma, and the like.

To solve the above technical problems, a twelfth aspect of the present invention provides a kit comprising the antibody according to the first aspect of the present invention, the bispecific antibody according to the second aspect of the present invention, the chimeric antigen receptor according to the sixth aspect of the present invention, the genetically modified cell according to the seventh aspect of the present invention, the antibody-drug conjugate according to the ninth aspect of the present invention, and/or the pharmaceutical composition according to the tenth aspect of the present invention, and optionally, instructions.

To solve the above technical problems, a thirteenth aspect of the present invention provides an administration device comprising: (1) an infusion module for administering to a subject in need thereof the pharmaceutical composition according to the tenth aspect of the present invention, and (2) optionally a pharmacodynamic monitoring module.

To solve the above technical problems, a fourteenth aspect of the present invention provides a method for detecting B7-H4, which comprises the step of detecting using the anti-B7-H4 antibody according to the first aspect of the present invention and/or the bispecific antibody according to the second aspect of the present invention.

Preferably, the method is for non-diagnostic and/or non-therapeutic purposes. Non-diagnostic and non-therapeutic purposes include, i.g., detecting B7-H4 contained in a test sample in the laboratory or confirming B7-H4 before medicine screening by using B7-H4 in drug development.

To solve the above technical problems, the present invention also provides a method for treating and/or preventing a tumor, which comprises administering to a subject in need thereof a therapeutically effective amount of the anti-B7-H4 antibody according to the first aspect of the present invention, the bispecific antibody according to the second aspect of the present invention, the chimeric antigen receptor according to the sixth aspect of the present invention, the genetically modified cell according to the seventh aspect of the present invention, the antibody-drug conjugate according to the ninth aspect of the present invention, and/or the pharmaceutical composition according to the tenth aspect of the present invention.

A therapeutically effective dose for the pharmaceutical composition of the present invention may be estimated initially in cell culture experiments or animal models such as rodents, rabbits, dogs, pigs, and/or primates. Animal models can also be used to determine the appropriate concentration range and route of administration, and subsequently an effective dose and a route of administration in humans. In general, the determination of and adjustment to the effective amount or dose to be administered and the assessment of when and how to make such adjustments are known to those skilled in the art.

To solve the above problems, the present invention also provides a combination therapy for treating cancer, which comprises administering to a patient in need thereof a therapeutically effective dose of a pharmaceutical composition and a combination therapeutic agent; the pharmaceutical composition is one according to the tenth aspect of the present invention.

Preferably, the combination therapeutic agent is one according to the tenth aspect of the present invention.

In the present application, unless otherwise defined, the scientific and technical terms used herein have the meanings generally understood by those skilled in the art. In addition, the laboratory operations of cell culture, molecular genetics, nucleic acid chemistry, and immunology used herein are the conventional procedures widely used in the corresponding fields. Meanwhile, to better understand the present invention, the definitions and explanations of the relevant terms are provided below.

In the present application, the term "variable" generally refers to the fact that certain portions of the sequences of the variable domains of antibodies vary considerably, resulting in the binding and specificity of various particular antibodies to their particular antigens. However, variability is not evenly distributed throughout the variable region of the antibody. It is mainly in three segments in each of the light chain and heavy chain variable regions called complementary determining regions (CDRs) or hypervariable regions (HVRs). The more highly conserved portions of the variable domains are called frameworks (FWRs). The variable domains of native heavy and light chains each comprise four FWRs largely in a β-sheet configuration. The FRs are connected by three CDRs to form a loop connection, and in some cases to form part of a β-sheet structure. The CDRs in each chain are held in close proximity by the FWRs and form, together with the CDRs from the other chain, antigen-binding sites of the antibody. The constant regions are not directly involved in the binding of the antibody to antigens, but they exhibit different effector functions, for example, being involved in antibody-dependent cellular cytotoxicity of the antibody.

The three-letter codes and single-letter codes for amino acids used in the present application are known to those skilled in the art, or are described in J. Biol. Chem, 243, p3558 (1968).

As used herein, the term "include/includes/including" or "comprise/comprises/comprising" is intended to mean that a composition and a method include the elements described but does not exclude other elements; but the case of "consist/consists/consisting of" is also included as the context dictates.

In the present application, the HCAb may be a full human antibody containing only "heavy chains" (a heavy chain-only antibody) produced from a transgenic mouse carrying an immune repertoire of human immunoglobulins-Harbour HCAb mouse (Harbour Antibodies BV, WO 2002/085945 A3), which is only half the size of conventional IgG antibodies and generally has only human antibody "heavy chain" variable domains and mouse Fc constant domains.

The term "antibody" described herein may include an immunoglobulin, which is of a tetrapeptide chain structure formed by connection between two identical heavy chains and two identical light chains by interchain disulfide bonds. Immunoglobulins differ in amino acid composition and arrangement of their heavy chain constant regions and therefore in their antigenicity. Accordingly, immunoglobulins can be classified into five classes, or isotypes of immunoglobulins, namely IgM, IgD, IgG, IgA, and IgE, with their corresponding heavy chains being the µ, δ, γ, α, and ε chains, respectively. The Ig of the same class can be divided into different subclasses according to the differences in amino acid composition of the hinge regions and the number and location of disulfide bonds in the heavy chains; for example, IgG can be divided into IgG1, IgG2, IgG3, and IgG4. Light chains are classified into κ or λ chains by the difference in the constant regions. Each of the five classes of Ig can have a κ chain or a λ chain.

In the present application, the light chain variable region of the antibody described herein may further comprise a light chain constant region comprising a human κ or λ chain or a variant thereof. In the present application, the heavy chain variable region of the antibody described herein may further comprise a heavy chain constant region comprising human IgG1, IgG2, IgG3 or IgG4 or a variant thereof.

In light chains and heavy chains, the variable region and constant region are linked by a "J" region of about 12 or more amino acids, and the heavy chain further comprises a "D" region of about 3 or more amino acids. Each heavy chain consists of a heavy chain variable region (VH) and a heavy chain constant region (CH). The heavy chain constant region consists of 3 domains (CH1, CH2, and CH3). Each light chain consists of a light chain variable region (VL) and a light chain constant region (CL). The light chain constant region consists of one domain CL. The constant region of the antibody can mediate the binding of immunoglobulins to host tissues or factors, including the binding of various cells of the immune system (e.g., effector cells) to the first component (C1q) of the classical complement system. The sequences of about 110 amino acids of the heavy and light chains of the antibody near the N-terminus vary considerably and thus are referred to as variable regions (V regions); the remaining amino acid sequences near the C-terminus are relatively stable and thus are referred to as constant regions (C regions). The variable region comprises 3 hypervariable regions (HVRs) and 4 framework regions (FWRs) with relatively conservative sequences. The 3 hypervariable regions determine the specificity of the antibody and thus are also referred to as complementary determining regions (CDRs). Each of the light chain variable regions (VLs) and the heavy chain variable regions (VHs) consists of 3 CDR regions and 4 FWR regions arranged from the amino-terminus to the carboxyl-terminus in the following order: FWR1, CDR1, FWR2, CDR2, FWR3, CDR3, and FWR4. The 3 CDR regions of the light chain refer to VLCDR1, VLCDR2, and VLCDR3; the 3 CDR regions of the heavy chain refer to VHCDR1, VHCDR2, and VHCDR3.

The term "human antibody" includes antibodies having variable and constant regions of human germline immunoglobulin sequences. The human antibody of the present application may include amino acid residues not encoded by human germline immunoglobulin sequences (e.g., mutations introduced by *in vitro* random or site-directed mutagenesis or *in vivo* somatic mutation). However, the term "human antibody" does not include antibodies in which CDR sequences derived from the germline of another mammalian species, such as a mouse, have been grafted into human framework sequences (i.e., "humanized antibodies").

As used herein, the term "specificity" with respect to an antibody means that an antibody recognizes a specific antigen but does not substantially recognize or bind to other molecules in a sample. For example, an antibody specifically binding to an antigen from one species may also bind to the antigen from one or more species. However, such interspecific cross-reactivity per se does not change the classification of antibodies by specificity. In another example, an antibody specifically binding to an antigen may also bind to the antigen in different allelic forms. However, such cross-reactivity per se does not change the classification of antibodies by specificity. In some cases, the term "specificity" or "specific binding" may be used to refer to the interaction of an antibody, a protein, or a peptide with a second chemical substance, meaning that the interaction is dependent on the presence of a particular structure (e.g., an antigenic determinant or epitope) in the chemical substance; for example, an antibody generally recognizes and binds to a particular protein structure rather than a protein. If an antibody has specificity for an epitope "A", the presence of a molecule containing the epitope A (or free, unlabeled A) will reduce the amount of labeled A bound to the antibody in a reaction containing labeled "A" and the antibody.

In the present application, the term "antigen-binding fragment" refers to an antigen-binding fragment and an antibody analog of the antibody, which generally include at least a portion of the antigen-binding region or variable region (e.g., one or more CDRs) of a parental antibody. The antigen-binding fragment retains at least some of the binding specificity of the parental antibody. Generally, the antigen-binding fragment retains at least 10% of the binding activity of the parental antibody when the activity is expressed on a molar basis. Preferably, the antigen-binding fragment retains at least 50%, 60%, 70%, 80%, 90%, 95%, 99%, or 100% or more of the binding affinity of the parental antibody for the target. Examples of the antigen-binding fragment include, but are not limited to: Fab, Fab', F(ab')₂, Fv fragments, linear antibodies, single-chain antibodies, nanobodies, single-domain antibodies, and multispecific antibodies. The antigen-binding fragment may be an engineered antibody variant. For a review of engineered antibody variants, see Holliger and Hudson (2005) Nat. Biotechnol. 23: 1126-1136.

The term "chimeric antigen receptor" or "CAR" used herein includes extracellular domains (extracellular binding domains), hinge domains, and transmembrane domains (transmembrane regions) capable of binding to antigens and polypeptides that pass a cytoplasmic signal to a domain (i.e., an intracellular signal domain). The hinge domain may be considered as a part for providing flexibility to an extracellular antigen-binding region. The intracellular signal domain refers to a protein that transmits information into a cell via a determined signaling pathway by generating a second messenger to regulate the activity of the cell, or a protein that functions as an effector by corresponding to such a messenger. It generates a signal that can promote the immune effector function of a cell of the CAR (e.g., a CART cell). The intracellular signal domain includes a signaling domain and may also include a co-stimulatory intracellular domain derived from a co-stimulatory molecule.

"Identity" or "mutation" refers to sequence similarity between two polynucleotide sequences or between two polypeptide sequences. When positions in two compared sequences are all occupied by the same base or amino acid monomer subunit, for example, if a position in each of two DNA molecules is occupied by adenine, the molecules are homologous at that position. The identity percentage between two sequences is a function of the number of matched or homologous positions shared by the two sequences divided by the number of the compared positions × 100%. For example, when sequences are optimally aligned, if 6 out of 10 positions in two sequences match or are homologous, the two sequences are 60% homologous. In general, the comparison is made when two aligned sequences give the greatest identity percentage.

The terms "polypeptide", "peptide", and "protein" (if single-stranded) are used interchangeably in the present application. The terms "nucleic acid", "nucleic acid sequence", "nucleotide sequence", or "polynucleotide sequence", and "polynucleotide" are used interchangeably.

The term "vector" used herein is a composition that comprises an isolated nucleic acid and is useful for delivering the isolated nucleic acid to the interior of a cell. Many vectors are known in the art, including but not limited to, linear polynucleotides, polynucleotides associated with ionic or amphiphilic compounds, plasmids, and viruses. Thus, the term "vector" includes autonomously replicating plasmids or viruses. The term should also be construed as including non-plasmid and non-viral compounds that facilitate the transfer of nucleic acids into cells, such as polylysine compounds and liposomes. Examples of viral vectors include, but are not limited to, adenoviral vectors, adeno-associated viral vectors, retroviral vectors, etc.

The expressions "cell" and "cell line" used in the present application are used interchangeably and all such designations include progeny. The term "host cell" refers to a cell to which a vector can be introduced, including, but not limited to, prokaryotic cells such as *E. coli,* fungal cells such as yeast cells, or animal cells such as fibroblasts, CHO cells, COS cells, NSO cells, HeLa cells, BHK cells, HEK 293 cells, or human cells.

The term "transfection" refers to the introduction of an exogenous nucleic acid into a eukaryotic cell. Transfection may be accomplished by a variety of means known in the art, including calcium phosphate-DNA co-precipitation, DEAE-dextran mediated transfection, polybrene-mediated transfection, electroporation, microinjection, liposome fusion, lipofection, protoplast fusion, retroviral infection, and biolistics.

The term "immune cell" refers to a cell that can elicit an immune response. The "immune cell" and other grammatical variations thereof may refer to an immune cell of any origin. The "immune cell" includes, for example, white blood cells (leukocytes) and lymphocytes (T cells, B cells, and natural killer (NK) cells) derived from hematopoietic stem cells (HSCs) produced in the bone marrow, and bone marrow-derived cells (neutrophils, eosinophils, basophils, monocytes, macrophages, and dendritic cells). The term "immune cell" may also refer to a human or non-human immune cell. For example, the immune cells may be derived from the blood, such as autologous T cells, allogeneic T cells, autologous NK cells, and allogeneic NK cells, or from cell lines, such as NK cell lines prepared by infection with the EBV virus, NK cells obtained by induced differentiation of embryonic stem cells and iPSCs, as well as NK92 cell lines.

As used herein, the term "T cell" refers to a class of lymphocytes that are matured in the thymus. T cells play an important role in cell-mediated immunity and are different from other lymphocytes (e.g., B cells) in that T cell receptors are present on the cell surface. "T cell" includes all types of immune cells expressing CD3, including T helper cells (CD4⁺ cells), cytotoxic T cells (CD8⁺ cells), natural killer T cells, T regulatory cells (Tregs), and γ-δT cells. "Cytotoxic cells" include CD8⁺ T cells, natural killer (NK) cells, and neutrophils, which are capable of mediating a cytotoxic response. As used herein, the term "NK cell" refers to a class of lymphocytes that originate in the bone marrow and play an important role in the innate immune system. NK cells provide a rapid immune response against virus-infected cells, tumor cells, or other stressed cells, even in the absence of antibodies and major histocompatibility complexes on the cell surface.

The term "optional", "optionally", "any", or "any one of" means that the event or circumstance subsequently described may, but not necessarily, occur, and that the description includes instances where the event or circumstance occurs or does not occur. For example, "optionally comprising 1 antibody heavy chain variable region" means that the antibody heavy chain variable region of a particular sequence may, but not necessarily, be present. As used herein, the "a" and "an" are used in the present invention to refer to one or more grammatical objects. Unless otherwise specifically stated in the content, the term "or" is used in the present invention to refer to, and is interchangeable with, the term "and/or". The "about" and "approximately" shall generally refer to an acceptable degree of error in the measured quantity in view of the nature or accuracy of the measurement. Exemplary degrees of error are typically within 10% thereof and more typically within 5% thereof. The method and composition disclosed in the present invention encompass polypeptides and nucleic acids having a specified sequence, a variant sequence, or a sequence substantially identical or similar thereto, e.g., a sequence that is at least 85%, 90%, 95%, 99% or more identical to the sequence specified. In the context of amino acid sequences, the term "substantially identical" is used in the present invention to refer to a first amino acid sequence.

As used herein, the term "pharmaceutically acceptable carrier" refers to a carrier that is pharmacologically and/or physiologically compatible with the subject and the active ingredient, e.g., any suitable physiologically or pharmaceutically acceptable auxiliary materials. It is well known in the art (see, e.g., *Remington's Pharmaceutical Sciences.* Edited by Gennaro AR, 19th ed. Pennsylvania: Mack Publishing Company, 1995) and includes, but is not limited to: pH adjusting agents, excipients, fillers, surfactants, adjuvants, ionic strength enhancers, diluents, osmotic pressure-maintaining agents, absorption-retarding agents, and preservatives. For example, pH adjusting agents include, but are not limited to, phosphate buffers. Surfactants include, but are not limited to, cationic, anionic, or nonionic surfactants, e.g., Tween-80. Ionic strength enhancers include, but are not limited to, sodium chloride. Preservatives include, but are not limited to, various antibacterial and antifungal agents, such as paraben, chloretone, phenol, and sorbic acid. Osmotic pressure-maintaining agents include, but are not limited to, sugars, NaCl, and analogs thereof. Absorption-retarding agents include, but are not limited to, monostearate salts and gelatin. Diluents include, but are not limited to, water, aqueous buffers (e.g., buffered saline), alcohols and polyols (e.g., glycerol), and the like. Preservatives include, but are not limited to, various antibacterial and antifungal agents, such as thiomersalate, 2-phenoxyethanol, paraben, chloretone, phenol, and sorbic acid. Stabilizers have the meaning commonly understood by those skilled in the art, and they are capable of stabilizing the desired activity of the active ingredient in a drug, and include, but are not limited to, sodium glutamate, gelatin, SPGA, sugars (such as sorbitol, mannitol, starch, sucrose, lactose, dextran, or glucose), amino acids (such as glutamic acid or glycine), proteins (such as dried whey, albumin, or casein) or degradation products thereof (such as lactalbumin hydrolysate), and the like. The pharmaceutically acceptable carrier of the present invention may be a conventional carrier in the art, and the pharmaceutical auxiliary material is a conventional pharmaceutical auxiliary material in the art.

As used herein, the term "EC₅₀" refers to the concentration for 50% of maximal effect, i.e., the concentration that can result in 50% of the maximal effect.

As used herein, the terms "cancer" and "tumor" are intended to include all types of cancerous growths or oncogenic processes, metastatic tissues or malignantly transformed cells, tissues or organs, regardless of their histopathological types or stages of invasiveness. Examples include, but are not limited to, solid tumors, hematologic cancer, soft tissue tumors, and metastatic lesions.

On the basis of the general knowledge in the art, the above preferred conditions can be combined arbitrarily to obtain preferred embodiments of the present invention.

The reagents and starting materials used in the present invention are commercially available.

The beneficial effects of the present invention are as follows:
1. The antibody of the present invention has the activity of binding to human B7-H4 and cynomolgus monkey B7-H4. In a certain preferred embodiment, the antibody is a novel fully human antibody containing only "heavy chains". It is only half the size of conventional IgG antibodies. Due to the absence of light chains, the antibody can be used for bispecific antibodies, and the problems of light chain mismatching and heterodimerization are solved.
2. When the antibody of the present invention is prepared into a B7-H4×CD3 bispecific antibody, the bispecific antibody still retains the activity of binding to human B7-H4 and cynomolgus monkey B7-H4, has strong killing effect on tumor cells, features low expression of induced nonspecific cytokines such as IL-6 and IFN-γ, and shows strong in vivo anti-tumor activity. In a certain preferred embodiment, the bispecific antibody has human Fc fragment in its structure and retains the binding of Fc to FcRn, thereby having a long half-life; meanwhile, a mutated (AAA) Fc is preferred to reduce binding to FcgR, thereby reducing non-specific T cell activation due to crosslinking of FcgR; a serial VHH form is adopted for the B7-H4 end, thus simplifying the mismatching of light chains and heavy chains and simultaneously maintaining good stability and hydrophilicity; the bivalent serial VHH increases the affinity for B7-H4 and also increases the selectivity for tumor cells with high expression of B7-H4; the activity of the CD3 end in the bispecific antibody is optimized and a medium-strength anti-CD3 antibody is adopted, so that the toxicity is reduced on the premise of ensuring the drug efficacy.

### BRIEF DESCRIPTION OF THE DRAWINGS

FIGs. 1A-1E show the binding of anti-human B7-H4 HCAb monoclonal antibodies at cellular level as assayed by FACS.
FIG. 2 shows the determination of affinity by the BLI method.
FIGs. 3A-3C show the structures of B7-H4×CD3 bispecific antibodies.
FIGs. 4A-4E show *in vitro* binding of B7-H4×CD3 bispecific antibodies on CHO-K1 cell strain overexpressing human, cynomolgus monkey, and mouse B7-H4 as assayed by FACS.
FIGs. 5A-5F show *in vitro* binding of B7-H4×CD3 bispecific antibodies on tumor cells endogenously expressing B7-H4 and T cells as assayed by FACS.
FIGs. 6A-6E show the results of the killing of MDA-MB-468 cells by T cells.
FIGs. 7A-7D show the results of the killing of OVCAR-3 cells by T cells.
FIGs. 8A-8C show the results of the killing of DLD-1 and MDA-MB-231 cells by T cells.
FIGs. 9A and 9B show the results of the induction of the nonspecific cytokine IL-6.
FIGs. 10A and 10B show the results of the induction of the nonspecific cytokine TNF-α.
FIGs. 11A and 11B show the results of the induction of the nonspecific cytokine IFN-γ.
FIGs. 12A-12C show *in vivo* drug efficacy experiments in mouse tumor models.

### DETAILED DESCRIPTION

The present invention is further illustrated by the following examples, which are not intended to limit the present invention. Experimental procedures without specified conditions in the following examples are performed in accordance with conventional procedures and conditions, or in accordance with instructions.

### Example 1. Acquisition of Anti-B7-H4 HCAB Antibody Molecules

The B7-H4 antigen can be used to immunize laboratory animals, which may be mice, rats, rabbits, sheep, camels, etc., to obtain antibody molecules specifically binding to B7-H4. Typically, the resulting antibody molecules are non-human antibodies. After obtaining non-human antibodies, these molecules need to be humanized by antibody engineering technology to reduce immunogenicity and improve druggability. However, the humanization of antibodies is complex in terms of the technology, and the humanized molecules tend to have reduced affinity for antigens. In addition, advances in transgenic technology have made it possible to develop genetically engineered mice that carry an immune repertoire of human immunoglobulins and have the endogenous murine immune repertoire deleted. The antibodies produced by the transgenic mice have fully human sequences, so that further humanization is not needed, and the efficiency of developing therapeutic antibodies is greatly improved. The Harbour HCAb mouse (Harbour Antibodies BV, WO 2002/085945 A3) is a transgenic mouse carrying an immune repertoire of human immunoglobulins and capable of producing novel "heavy chain"-only antibodies that are only half the size of conventional IgG antibodies. The antibodies produced have only human antibody "heavy chain" variable domains and mouse Fc constant domains. Due to the absence of light chains, this antibody almost solves the problems of light chain mismatching and heterodimerization, allowing the technical platform to develop products that are difficult to realize by a conventional antibody platform.

### 1.1 Immunization of mice

HCAB mice were immunized with HEK293T cells overexpressing human B7-H4 (HEK293T/hu B7-H4, Kyinno Biotechnology), and each mouse was immunized with 5 × 10⁶ cells each time via intraperitoneal injection. In each round of immunization, each mouse received a total injection dose of 100 µL. The interval between rounds of booster immunization was at least two weeks. In general, there are 6-7 rounds of booster immunizations. The immunization was performed at days 0, 14, 28, 42, 56, 70, 84, and 96; and the antibody titer in serum of mice was measured at days 49 and 77. The last round of booster immunization was performed 5 days before the isolation of HCAB mouse splenic B cells.

### 1.2 Serum titer assay

At specific time points, the serum of mice was collected, and the titer of antibody binding to B7-H4 protein in the serum was determined by the ELISA method and the titer of antibody binding to B7-H4-overexpressing cells in the serum was determined by the FACS method.

In the ELISA method, an ELISA plate (corning, 9018) was coated with 1 µg/mL hB7-H4-ECD-his protein (Sino Biological, # 10738-H08H) at 100 µL/well and incubated overnight at 4 °C; after 2 rinses, the plate was blocked with PBST containing 1% BSA for 2 h at 37 °C; then the serially diluted serum was added at 100 µL/well and the plate was incubated for 1 h at 37 °C; after 3 rinses, the anti-mouse-HRP (Bethyl, Cat# A90-231P) diluted at 1:5000 was added at 100 µL/well and the plate was incubated for 30 min at 37 °C. After 3 rinses, the TMB substrate was added at 100 µL/well and the plate was incubated for about 10 min, and then 1 N HCl was added at 50 µL/well for termination of the color development, and then the absorbance at 450 nm was read (Molecular Devices, Plus 384).

In the FACS method, serially diluted mouse serum was incubated with HEK293T/hu B7-H4 cells for 1 h at 4 °C; after 2 washes of the cells, a secondary antibody Goat Anti-Mouse IgG (H+L) (Jackson, Cat# 115-605-062) was added and the cells were incubated for 1 h at 4 °C; after 2 washes, the cells were resuspended and detected by a flow cytometer (ACEA Novocyte3000). HEK293T cells served as background controls.

### 1.3 Acquisition of HCAb monoclones and antibody sequences by phage display technology

### a. Construction of phage library

Spleen cells were collected from the immunized mice in Example 1.1, and splenic B cells were isolated therefrom. After RNA was extracted by TRIZOl with reference to the product instructions (Thermofisher, Cat. No. 15596018), RT-PCR was performed with reference to the product instructions (Thermofisher, Cat. No. 11756500). VHH fragments were obtained by PCR amplification using cDNA as the template.

The PCR amplification system is shown in the table below:

| Total RNA-cDNA | 1 µL/1 µL H2O as a negative control |
|---|---|
| 5× Q5 reaction buffer | 10µL |
| 2.5mM dNTP | 4µL |
| Forward primer (10 µM) | 2µL |
| Reverse primer (10 µM) | 2µL |
| Q5 DNA polymerase | 0.5µL |
| ddH2O | 30.5µL |
| Total volume | 50µL |

The PCR amplification procedure is shown in the table below:

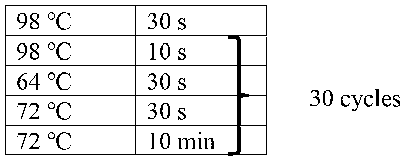

The resulting ligation products were transformed into SS320 (Lucigen, Cat. No. 60512-1), and a phage library was prepared. Three rounds of bio-panning (the steps are described below) were performed, and screening was performed by FACS with biotinylated B7-H4. All the selected candidates (hits) were sent for sequencing (the steps are described below), and specific clones were produced.

### b. Bio-panning

Non-specifically bound molecules were removed from the phage library (1E13 phage particles) by using streptavidin (SA)-coated beads (Thermofisher, Cat.No.11206), and the phages were incubated with SA-Bio-B7-H4 beads (see product instructions) for 1 h at room temperature, followed by washing 10 times with 1 × PBST. After washing, a citrate buffer at pH 3.0 was added to the bead/phage mixture, and the resulting mixture was incubated at room temperature for 10 min. Then, a Tris-HCl neutralization buffer at pH 9.0 was added, and the mixture was used to infect SS320 cells at 37 °C for 45 min. Then, 50 µL of M13K07 helper phages (NEB, Cat. No. N0315S) at 2E12/mL was added to the infected cells, and finally fresh 2× YT (containing 100 µg/mL Amp, 50 µg/mL Kan, and 1 mM IPTG) was added, and the mixture was incubated at 30 °C overnight. The overnight culture was centrifuged, and the supernatant was collected. 20% PEG6000/2.5 M NaCl was added to the supernatant (1:5, volume ratio), and the mixture was centrifuged at 8000 rpm for 20 min at 4 °C. The phage pellet obtained after centrifugation was resuspended in 1 mL of 1× PBS. The resulting supernatant (phage particles) was transferred to a new tube for the second round of panning. The procedures of the second and third rounds of panning were the same as the previous steps.

### c. Preliminary screening

Colonies were picked out and added into a 96-well plate containing YT/Amp medium (Sangon Biotech, Cat. No. A507016-0250) for growing at 37 °C for 3 h. IPTG was added to give a final concentration of 1 mM, and the mixture was induced at 30 °C overnight. The supernatant was then subjected to centrifugal sedimentation, and the resulting supernatant was subjected to an FACS assay. Clones with good binding to human and monkey B7-H4 were selected for sequencing.

### 1.4 Acquisition of anti-B7-H4 H2L2 antibody molecules

The B7-H4 recombinant protein or the cell overexpressing B7-H4 is used to immunize laboratory animals, which may be mice, rats, rabbits, sheep, camels, etc., to obtain antibody molecules specifically binding to B7-H4. Typically, the resulting antibody molecules are non-human antibodies. After obtaining non-human antibodies, these molecules need to be humanized by antibody engineering technology to reduce immunogenicity and improve druggability. However, the humanization of antibodies is complex in terms of the technology, and the humanized molecules tend to have reduced affinity for antigens. In addition, advances in transgenic technology have made it possible to develop genetically engineered mice that carry an immune repertoire of human immunoglobulins and have the endogenous murine immune repertoire deleted. The Harbour H2L2 mice (Harbour Antibodies BV) are transgenic mice that carry an immune repertoire of human immunoglobulins, and the antibodies generated by the transgenic mice have fully human sequences, so that further humanization is not needed, and the efficiency of developing therapeutic antibodies is greatly improved.

Then, anti-B7-H4 antibodies were screened by *in vitro* cloning technique for B cells, and the specific procedures are as follows:
The spleens of the mice were taken out, ground, and filtered through a 200-mesh filter, and the single cell suspensions were sorted according to the mouse memory B cell sorting kit (Miltenyi, #130-095-838). The cells obtained by sorting were subjected to immunofluorescence staining.

B200 positive cells (BioLegend, #103227), IgM negative cells (BioLegend, #406506), and B7-H4 specific positive cells (BioLegend, #405207) were sorted using a flow cell sorter S3e. The cells obtained by sorting were cultured in a 96-well cell culture plate at a density of 5 cells per well, and irradiated EL4 cells were previously plated on the cell culture plate as feeder cells.

After 14 days of culture, culture supernatants were collected and subjected to ELISA assay, and for wells having binding activity for B7-H4 protein, cells were taken out and subjected to RT-PCR (SMART-Seq v4 Ultra Low Input RNA Kit for Sequencing (#634892), I-5^{™} 2× High-Fidelity Master Mix (#I5HM-5000)). The light and heavy chains obtained by amplification were spliced into a scFv by overlap PCR and expressed in *E. coli,* the expression supernatants were subjected to ELISA assay, and the positive clones were sequenced.

### 1.5 Sequence analysis and sequence optimization of anti-B7-H4 antibodies

Positive clones obtained by screening were used to prepare recombinant antibodies PR006004 and PR006008 according to the method described in Example 1.6. In this example, the sequences of the variable domains of the anti-B7-H4 monoclonal antibody molecules obtained from immunized Harbour HCAb mice were human antibody sequences. The germline gene analysis and post-translational modification site (PTM) analysis of the sequences are listed in Table 1-1.

PR006004 and PR006008 contained a possible site of aspartic acid isomerization (DG) in CDR2. To reduce the risk of this potential post-translational modification site (PTM), DG was mutated to DS, resulting in new molecules PR007440 and PR007441, respectively (Table 1-2).

Further, to lower the isoelectric point of PR006004, basic amino acids of CDR regions were selected for random mutation based on antibody sequence analysis. Then, screening was performed using FACS binding experiments, and the selected candidate molecules were subjected to expression, purification, and identification. The sequences of the new antibody molecules obtained from amino acid mutations on the sequences of antibody PR006004 are listed in Table 1-2.

**Table 1-1. Initial antibody**

| **Clone No.** | **VH germline V gene** | **VH PTM** | **Recombinant antibody** | **Recombinant antibody subtype** |
|---|---|---|---|---|
| R7004M1A2 | VH3-74*01 | DG (HCDR2) | PR006004 | IgG1 |
| R7004M6D4 | VH3-74*01 | DG (HCDR2) | PR006008 | IgG1 |

**Table 1-2. PTM and charge mutations**

| **Initial antibody** | **Variant** | **Variable region mutation** | **Recombinant antibody subtype** |
|---|---|---|---|
| PR006004 | PR007440 | G55S | IgG1 |
| PR006008 | PR007441 | G55S | IgG1 |
| PR006004 | PR007195 | V46E, G55S, R56D, A61E | IgG1 |
| PR006004 | PR007196 | V46E, G55S, R56E | IgG1 |
| PR006004 | PR007200 | V46E, G55S, R56S | IgG1 |
| PR006004 | PR007201 | V46E, G55S, R56S, A61E | IgG1 |
| PR006004 | PR007202 | V46E, G55S, R56T | IgG1 |
| PR006004 | PR007203 | V46E, G55S, R56T, A61E | IgG1 |

### 1.6 Preparation of anti-B7-H4 fully human recombinant antibodies

Mammalian host cells (e.g., human embryonic kidney cell HEK293) were transfected with the plasmids encoding the antibody heavy chains, and purified anti-B7-H4 recombinant heavy-chain antibodies could be obtained using conventional recombinant protein expression and purification techniques. Specifically, HEK293 cells were expanded in FreeStyle^{™} F17 Expression Medium (Thermo, A1383504). Before the transient transfection, the cells were adjusted to a concentration of 6× 10E+05 cells/mL and cultured in a shaker at 37 °C and 8% CO₂ for 24 h to make a concentration of 1.2×10E+06 cells/mL. 30 mL of the cultured cells were prepared, and 30 µg of the above plasmids encoding heavy chains was dissolved in 1.5 mL of Opti-MEM reduced serum medium (Thermo, 31985088). Then 120 µL of 1 mg/mL PEI (Polysciences, Inc, Cat# 23966-2) was dissolved in 1.5 mL of Opti-MEM, and the mixture was left to stand for 5 min. PEI was slowly added to the plasmid, and the mixture was incubated at room temperature for 10 min. The mixed solution of plasmid and PEI was slowly added dropwise while shaking the culture flask, and the cells were cultured in a shaker at 37 °C and 8% CO₂ for 5 days. Cell viability was measured after 5 days. The culture was collected and centrifuged at 3300 g for 10 min, and then the supernatant was collected and centrifuged at high speed to remove impurities. A gravity column (Bio-Rad, #7311550) containing MabSelect^{™} (GE Healthcare Life Science, Cat#71-5020-91 AE) was equilibrated with PBS (pH 7.4) and rinsed with 2-5 column volumes of PBS. The supernatant sample was loaded onto the column. The column was rinsed with 5-10 column volumes of PBS. The target protein was eluted with 0.1 M glycine (pH 3.5). The eluate was adjusted to neutrality with Tris-HCl (pH 8.0) and concentrated and buffer exchanged into PBS buffer with an ultrafiltration tube (Millipore, UFC901024) to obtain a purified anti-B7-H4 heavy-chain antibody solution.

### 1.7 Analysis of protein purity and polymers by SEC-HPLC

In this example, analytical size-exclusion chromatography (SEC) was used to analyze the protein sample for purity and polymer form. An analytical chromatography column TSKgel G3000SWxl (Tosoh Bioscience, #08541, 5 µm, 7.8 mm × 30 cm) was connected to a high-pressure liquid chromatograph HPLC (Agilent Technologies, Agilent 1260 Infinity II) and equilibrated with a PBS buffer at room temperature for at least 1 h. A proper amount of the protein sample (at least 10 µg) was filtered through a 0.22 µm filter membrane and then injected into the system, and a HPLC program was set: the sample was eluted in the chromatography column with a PBS buffer at a flow rate of 1.0 mL/min for a maximum of 25 min. An analysis report was generated by the HPLC, with the retention time of the components with different molecular sizes in the sample reported.

### 1.8 Analysis of protein purity and hydrophobicity by HPLC-HIC

Analytical hydrophobic interaction chromatography (HIC) was used to analyze the protein sample for purity and hydrophobicity. An analytical chromatography column TSKge1 Buty1-NPR (Tosoh Bioscience, 14947, 4.6 mm × 3.5 cm) was connected to a high-pressure liquid chromatograph (HPLC, model: Agilent Technologies, Agilent 1260 Infinity II) and equilibrated with a PBS buffer at room temperature for at least 1 h. The program for HPLC was set: a linear gradient from 100% mobile phase A (20 mM histidine, 1.8 M ammonium sulfate, pH 6.0) to 100% mobile phase B (20 mM histidine, pH 6.0) within 16 min; flow rate: 0.7 mL/min; protein sample concentration: 1 mg/mL; and injection volume: 20 µL. The detection wavelength was 280 nm. After being recorded, the chromatogram was integrated using ChemStation software and relevant data were calculated. An analysis was generated, with the retention time of the components with different molecular sizes in the sample reported.

### 1.9 Determination of thermostability of protein molecules by DSF or UNcle

Differential scanning fluorimetry (DSF) is a commonly used high-throughput method for determining the thermostability of proteins. In this method, changes in the fluorescence intensity of the dye binding to unfolded protein molecules were monitored using a real-time quantitative fluorescence PCR instrument to reflect the denaturation process of the protein and thus to reflect the thermostability of the protein molecule. In this example, the thermal denaturation temperature (Tm) of a protein molecule was measured by DSF. 10 µg of protein was added to a 96-well PCR plate (Thermo, #AB-0700/W), followed by the addition of 2 µL of 100× diluted dye SYPROTM (Invitrogen, #2008138), and then the mixture in each well was brought to a final volume of 40 µL by adding buffer. The PCR plate was sealed, placed in a real-time quantitative fluorescence PCR instrument (Bio-Rad CFX96 PCR System), and incubated first at 25 °C for 5 min, and then the temperature gradually increased from 25 °C to 95 °C at a gradient of 0.2 °C/0.2 min, and the temperature decreased to 25 °C at the end of the test. The FRET scanning mode was used and data analysis was performed using Bio-Rad CFX Maestro software to calculate the Tm of the sample.

Unchained Labs (Uncle) is a multifunctional one-stop protein stability analysis platform that characterizes protein stability by total fluorescence, static light scattering (SLS), and dynamic light scattering (DLS) assay methods. The parameters of melting temperature (Tm), aggregation temperature (Tagg), and particle size (diameter) can be obtained simultaneously for the same group of samples. In this example, a "Tm & Tagg with optional DLS" application of Uncle was selected for the determination. 9 µL of the sample was added to a Uni tube, and the temperature was set to gradually increase from 25 °C to 95 °C at a gradient of 0.3 °C/min. Four acquisitions of data were performed at the beginning and end of DLS assay, each for 5 s. After the experiment was finished, the Tm value of each sample was calculated according to a barycentric mean (BCM) formula using the Uncle analysis software.

The expression information and physicochemical properties of the obtained antibodies are shown in Table 1-3.

**Table 1-3. Expression and physicochemical properties of antibodies**

| Antibody | Yield after one-step purification (mg/L) | SEC-HPLC purity (%) | HIC-HPLC retention time (min) | Corresponding ammonium sulfate concentration (M) | Tmi (°C) | Determination method for Tm | PI |
|---|---|---|---|---|---|---|---|
| PR006004 | 40.31 | 98.74 | 17.127 | 0.66 | 61.4 | DSF | 8.82 |
| PR006008 | 51.7 | 100 | 16.193 | 0.78 | 59.4 | DSF | 7.69 |
| PR007440 | 123.5 | 95.756 | | | | | 8.82 |
| PR007441 | 16.4 | 100 | | | | | 7.69 |
| PR007195 | 21.29 | 72.71 | 17.625 | 0.6 | 45.6 | DSF | 6.93 |
| PR007196 | 60.82 | 97.45 | | | | | 7.2 |
| PR007200 | 45.93 | 97.68 | 17.81 | 0.58 | 59.4 | DSF | 7.59 |
| PR007201 | 43.47 | 92.1 | 17.662 | 0.6 | 52 | DSF | 7.2 |
| PR007202 | 44.67 | 96.79 | 17.844 | 0.58 | 59.2 | DSF | 7.59 |
| PR007203 | 50.53 | 90.74 | 17.65 | 0.6 | 52 | DSF | 7.2 |
| PR007354 | 150 | 89.19 | | | | | 9.23 |
| PR007355 | 202.9 | 95.93 | | | | | 9.11 |
| PR006391 | 94.5 | 100 | 17.397 | 0.64 | 54.6 | Uncle | 9.34 |
| PR006407 | 62.5 | 98.8 | 16.834 | 0.71 | 47.6 | Uncle | 9.14 |
| PR006840 | 59.9 | 98.97 | | | | | 9.25 |
| PR007077 | 33 | 93.984 | 17.9 | 0.59 | 57 | Uncle | 9.34 |
| PR007078 | 34 | 68.496 | 17.3 | 0.65 | 56.1 | Uncle | 9.14 |
| PR007168 | 43.8 | 99.077 | | | | | 9.25 |
| PR005885 | 108.4 | 99.18 | 17.39 | 0.64 | 57 | DSF | 9.26 |

### 1.10 Anti-B7-H4 antibody sequences and numbers

In the present invention, the amino acid sequences of the listed CDRs are shown according to the Chothia scheme. However, it is well known to those skilled in the art that the CDRs of an antibody can be defined in the art using a variety of methods, such as the Kabat scheme based on sequence variability (see Kabat et al., Sequences of Proteins of Immunological Interest, Fifth Edition, National Institutes of Health (U.S.), Bethesda, Maryland (1991)), and the Chothia scheme based on the location of the structural loop regions (see J Mol Biol 273: 927-48, 1997). In the technical solution of the present invention, the Combined scheme comprising the Kabat scheme and the Chothia scheme can also be used to determine the amino acid residues in a variable domain sequence. The Combined scheme combines the Kabat scheme with the Chothia scheme to obtain a larger range. See Table 4 for details. It will be understood by those skilled in the art that unless otherwise specified, the terms "CDR" and "complementary determining region" of a given antibody or a region (e.g., variable region) thereof are construed as encompassing complementary determining regions as defined by any one of the above known schemes described herein. Although the scope claimed in the present invention is the sequences shown based on the Chothia scheme, the amino acid sequences corresponding to the other schemes for numbering CDRs shall also fall within the scope of the present invention.

**Table 1-4. Schemes for defining CDRs of antibodies of the present application**

| | Kabat | Chothia | Combined |
|---|---|---|---|
| HCDR1 | H31--H35 | H26--H32 | H26-H35 |
| HCDR2 | H50--H65 | H52--H56 | H50-H65 |
| HCDR3 | H95--H102 | H95--H102 | H95-H102 |

In the table, Haa-Hbb can refer to an amino acid sequence from position aa (Chothia numbering scheme) to position bb (Chothia numbering scheme) beginning at the N-terminus of the heavy chain of the antibody. For example, H26-H35 can refer to an amino acid sequence from position 26 to position 35 beginning at the N-terminus of the heavy chain of the antibody according to the Chothia numbering scheme. It should be known to those skilled in the art that there are positions where insertion sites are present when numbering CDRs with the Chothia scheme (see http://bioinf.org.uk/abs/).

Sequence numbers corresponding to the sequences of the anti-B7-H4 antibodies of the present invention are listed in Table 1-5, Table 1-6, Table 1-7, and Table 1-8.

**Table 1-5**

| **Antibody No.** | **HCDR1** | | **HCDR2** | | **HCDR3** | |
|---|---|---|---|---|---|---|
| | SEQ | Amino acid sequence | SEQ | Amino acid sequence | SEQ | Amino acid sequence |
| PR006004 | 4 | GFAFSNY | 11 | SGDGRS | 24 | DRFGDDYYYGMNV |
| PR006008 | 5 | GFTFTDF | 12 | SPDGSS | 25 | FSTGWHRIEYFQH |
| PR007440 | 4 | GFAFSNY | 17 | SGDSRS | 24 | DRFGDDYYYGMNV |
| PR007441 | 5 | GFTFTDF | 18 | SPDSSS | 25 | FSTGWHRIEYFQH |
| PR007195 | 4 | GFAFSNY | 13 | SGDSDS | 24 | DRFGDDYYYGMNV |
| PR007196 | 4 | GFAFSNY | 14 | SGDSES | 24 | DRFGDDYYYGMNV |
| PR007200 | 4 | GFAFSNY | 15 | SGDSSS | 24 | DRFGDDYYYGMNV |
| PR007201 | 4 | GFAFSNY | 15 | SGDSSS | 24 | DRFGDDYYYGMNV |
| PR007202 | 4 | GFAFSNY | 16 | SGDSTS | 24 | DRFGDDYYYGMNV |
| PR007203 | 4 | GFAFSNY | 16 | SGDSTS | 24 | DRFGDDYYYGMNV |

**Table 1-6**

| **Antibody No.** | **Heavy chain** | **VH** | | **FWR 1** | **FWR 2** | **FWR 3** | **FW R4** |
|---|---|---|---|---|---|---|---|
| | | SEQ | Amino acid sequence | | | | |
| PR006004 | 48 | 36 | EVQLVESGGGLVQPGGSLRLSCAASGFAFSN YWMHWARQVPGKGRVWISRISGDGRSTSY ADSVKGRFTISRDNAKNMVYLQMNNLRAE DTAVYYCARDRFGDDYYYGMNVWGQGTT VTVSS | 2 | 7 | 20 | 27 |
| PR006008 | 49 | 37 | EVQLVESGGGLVQPGGSLRLSCAASGFTFTD FWMHWVRQVPGKGREWVSRISPDGSSTSY EDSVKGRFTISRDNAKNTVYLQMHGLRAE DTAVYYCTRFSTGWHRIEYFQHWGQGTLVT VSS | 2 | 8 | 21 | 26 |
| PR007440 | 56 | 44 | EVQLVESGGGLVQPGGSLRLSCAASGFAFSN YWMHWARQVPGKGRVWISRISGDSRSTSY ADSVKGRFTISRDNAKNMVYLQMNNLRAE DTAVYYCARDRFGDDYYYGMNVWGQGTT VTVSS | 2 | 7 | 20 | 27 |
| PR007441 | 57 | 45 | EVQLVESGGGLVQPGGSLRLSCAASGFTFTD FWMHWVRQVPGKGREWVSRISPDSSSTSY EDSVKGRFTISRDNAKNTVYLQMHGLRAE DTAVYYCTRFSTGWHRIEYFQHWGQGTLVT VSS | 2 | 8 | 21 | 26 |
| PR007195 | 50 | 38 | EVQLVESGGGLVQPGGSLRLSCAASGFAFSN YWMHWARQVPGKGREWISRISGDSDSTSY EDSVKGRFTISRDNAKNMVYLQMNNLRAE DTAVYYCARDRFGDDYYYGMNVWGQGTT VTVSS | 2 | 9 | 22 | 27 |
| PR007196 | 51 | 39 | EVQLVESGGGLVQPGGSLRLSCAASGFAFSN YWMHWARQVPGKGREWISRISGDSESTSYA DSVKGRFTISRDNAKNMVYLQMNNLRAED TAVYYCARDRFGDDYYYGMNVWGQGTTV TVSS | 2 | 9 | 20 | 27 |
| PR007200 | 52 | 40 | EVQLVESGGGLVQPGGSLRLSCAASGFAFSN YWMHWARQVPGKGREWISRISGDSSSTSYA DSVKGRFTISRDNAKNMVYLQMNNLRAED TAVYYCARDRFGDDYYYGMNVWGQGTTV TVSS | 2 | 9 | 20 | 27 |
| PR007201 | 53 | 41 | EVQLVESGGGLVQPGGSLRLSCAASGFAFSN YWMHWARQVPGKGREWISRISGDSSSTSYE DSVKGRFTISRDNAKNMVYLQMNNLRAED TAVYYCARDRFGDDYYYGMNVWGQGTTV TVSS | 2 | 9 | 22 | 27 |
| PR007202 | 54 | 42 | EVQLVESGGGLVQPGGSLRLSCAASGFAFSN YWMHWARQVPGKGREWISRISGDSTSTSYA DSVKGRFTISRDNAKNMVYLQMNNLRAED TAVYYCARDRFGDDYYYGMNVWGQGTTV TVSS | 2 | 9 | 20 | 27 |
| PR007203 | 55 | 43 | EVQLVESGGGLVQPGGSLRLSCAASGFAFSN YWMHWARQVPGKGREWISRISGDSTSTSYE DSVKGRFTISRDNAKNMVYLQMNNLRAED TAVYYCARDRFGDDYYYGMNVWGQGTTV TVSS | 2 | 9 | 22 | 27 |

**Table 1-7**

| **Antibody No**. | **Heavy chain** | |
|---|---|---|
| | SEQ | Amino acid sequence |
| PR006004 | 48 | EVQLVESGGGLVQPGGSLRLSCAASGFAFSNYWMHWARQVPGKGRVWISRISG DGRSTSYADSVKGRFTISRDNAKNMVYLQMNNLRAEDTAVYYCARDRFGDDY YYGMNVWGQGTTVTVSSEPKSSDKTHTCPPCPAPELLGGPSVFLFPPKPKDTLMI SRTPEVTCVVVDVSHEDPEVKFNWYVDGVEVHNAKTKPREEQYNSTYRVVSVL TVLHQDWLNGKEYKCKVSNKALPAPIEKTISKAKGQPREPQVYTLPPSREEMTK NQVSLTCLVKGFYPSDIAVEWESNGQPENNYKTTPPVLDSDGSFFLYSKLTVDKS RWQQGNVFSCSVMHEALHNHYTQKSLSLSPGK |
| PR006008 | 49 | EVQLVESGGGLVQPGGSLRLSCAASGFTFTDFWMHWVRQVPGKGREWVSRISP DGSSTSYEDSVKGRFTISRDNAKNTVYLQMHGLRAEDTAVYYCTRFSTGWHRIE YFQHWGQGTLVTVSSEPKSSDKTHTCPPCPAPELLGGPSVFLFPPKPKDTLMISRT PEVTCVVVDVSHEDPEVKFNWYVDGVEVHNAKTKPREEQYNSTYRVVSVLTVL HQDWLNGKEYKCKVSNKALPAPIEKTISKAKGQPREPQVYTLPPSREEMTKNQV SLTCLVKGFYPSDIAVEWESNGQPENNYKTTPPVLDSDGSFFLYSKLTVDKSRWQ QGNVFSCSVMHEALHNHYTQKSLSLSPGK |
| PR007440 | 56 | EVQLVESGGGLVQPGGSLRLSCAASGFAFSNYWMHWARQVPGKGRVWISRISG DSRSTSYADSVKGRFTISRDNAKNMVYLQMNNLRAEDTAVYYCARDRFGDDYY YGMNVWGQGTTVTVSSEPKSSDKTHTCPPCPAPELLGGPSVFLFPPKPKDTLMIS RTPEVTCVVVDVSHEDPEVKFNWYVDGVEVHNAKTKPREEQYNSTYRVVSVLT VLHQDWLNGKEYKCKVSNKALPAPIEKTISKAKGQPREPQVYTLPPSREEMTKN QVSLTCLVKGFYPSDIAVEWESNGQPENNYKTTPPVLDSDGSFFLYSKLTVDKSR WQQGNVFSCSVMHEALHNHYTQKSLSLSPGK |
| PR007441 | 57 | EVQLVESGGGLVQPGGSLRLSCAASGFTFTDFWMHWVRQVPGKGREWVSRISP DSSSTSYEDSVKGRFTISRDNAKNTVYLQMHGLRAEDTAVYYCTRFSTGWHRIE YFQHWGQGTLVTVSSEPKSSDKTHTCPPCPAPELLGGPSVFLFPPKPKDTLMISRT PEVTCVVVDVSHEDPEVKFNWYVDGVEVHNAKTKPREEQYNSTYRVVSVLTVL HQDWLNGKEYKCKVSNKALPAPIEKTISKAKGQPREPQVYTLPPSREEMTKNQV SLTCLVKGFYPSDIAVEWESNGQPENNYKTTPPVLDSDGSFFLYSKLTVDKSRWQ QGNVFSCSVMHEALHNHYTQKSLSLSPGK |
| PR007195 | 50 | EVQLVESGGGLVQPGGSLRLSCAASGFAFSNYWMHWARQVPGKGREWISRISG DSDSTSYEDSVKGRFTISRDNAKNMVYLQMNNLRAEDTAVYYCARDRFGDDYY YGMNVWGQGTTVTVSSEPKSSDKTHTCPPCPAPELLGGPSVFLFPPKPKDTLMIS RTPEVTCVVVDVSHEDPEVKFNWYVDGVEVHNAKTKPREEQYNSTYRVVSVLT VLHQDWLNGKEYKCKVSNKALPAPIEKTISKAKGQPREPQVYTLPPSREEMTKN QVSLTCLVKGFYPSDIAVEWESNGQPENNYKTTPPVLDSDGSFFLYSKLTVDKSR WQQGNVFSCSVMHEALHNHYTQKSLSLSPGK |
| PR007196 | 51 | EVQLVESGGGLVQPGGSLRLSCAASGFAFSNYWMHWARQVPGKGREWISRISG DSESTSYADSVKGRFTISRDNAKNMVYLQMNNLRAEDTAVYYCARDRFGDDYY YGMNVWGQGTTVTVSSEPKSSDKTHTCPPCPAPELLGGPSVFLFPPKPKDTLMIS RTPEVTCVVVDVSHEDPEVKFNWYVDGVEVHNAKTKPREEQYNSTYRVVSVLT VLHQDWLNGKEYKCKVSNKALPAPIEKTISKAKGQPREPQVYTLPPSREEMTKN QVSLTCLVKGFYPSDIAVEWESNGQPENNYKTTPPVLDSDGSFFLYSKLTVDKSR WQQGNVFSCSVMHEALHNHYTQKSLSLSPGK |
| PR007200 | 52 | EVQLVESGGGLVQPGGSLRLSCAASGFAFSNYWMHWARQVPGKGREWISRISG DSSSTSYADSVKGRFTISRDNAKNMVYLQMNNLRAEDTAVYYCARDRFGDDYY YGMNVWGQGTTVTVSSEPKSSDKTHTCPPCPAPELLGGPSVFLFPPKPKDTLMIS RTPEVTCVVVDVSHEDPEVKFNWYVDGVEVHNAKTKPREEQYNSTYRVVSVLT VLHQDWLNGKEYKCKVSNKALPAPIEKTISKAKGQPREPQVYTLPPSREEMTKN QVSLTCLVKGFYPSDIAVEWESNGQPENNYKTTPPVLDSDGSFFLYSKLTVDKSR WQQGNVFSCSVMHEALHNHYTQKSLSLSPGK |
| PR007201 | 53 | EVQLVESGGGLVQPGGSLRLSCAASGFAFSNYWMHWARQVPGKGREWISRISG DSSSTSYEDSVKGRFTISRDNAKNMVYLQMNNLRAEDTAVYYCARDRFGDDYY YGMNVWGQGTTVTVSSEPKSSDKTHTCPPCPAPELLGGPSVFLFPPKPKDTLMIS RTPEVTCVVVDVSHEDPEVKFNWYVDGVEVHNAKTKPREEQYNSTYRVVSVLT VLHQDWLNGKEYKCKVSNKALPAPIEKTISKAKGQPREPQVYTLPPSREEMTKN QVSLTCLVKGFYPSDIAVEWESNGQPENNYKTTPPVLDSDGSFFLYSKLTVDKSR WQQGNVFSCSVMHEALHNHYTQKSLSLSPGK |
| PR007202 | 54 | EVQLVESGGGLVQPGGSLRLSCAASGFAFSNYWMHWARQVPGKGREWISRISG DSTSTSYADSVKGRFTISRDNAKNMVYLQMNNLRAEDTAVYYCARDRFGDDYY YGMNVWGQGTTVTVSSEPKSSDKTHTCPPCPAPELLGGPSVFLFPPKPKDTLMIS RTPEVTCVVVDVSHEDPEVKFNWYVDGVEVHNAKTKPREEQYNSTYRVVSVLT VLHQDWLNGKEYKCKVSNKALPAPIEKTISKAKGQPREPQVYTLPPSREEMTKN QVSLTCLVKGFYPSDIAVEWESNGQPENNYKTTPPVLDSDGSFFLYSKLTVDKSR WQQGNVFSCSVMHEALHNHYTQKSLSLSPGK |
| PR007203 | 55 | EVQLVESGGGLVQPGGSLRLSCAASGFAFSNYWMHWARQVPGKGREWISRISG DSTSTSYEDSVKGRFTISRDNAKNMVYLQMNNLRAEDTAVYYCARDRFGDDYY YGMNVWGQGTTVTVSSEPKSSDKTHTCPPCPAPELLGGPSVFLFPPKPKDTLMIS RTPEVTCVVVDVSHEDPEVKFNWYVDGVEVHNAKTKPREEQYNSTYRVVSVLT VLHQDWLNGKEYKCKVSNKALPAPIEKTISKAKGQPREPQVYTLPPSREEMTKN QVSLTCLVKGFYPSDIAVEWESNGQPENNYKTTPPVLDSDGSFFLYSKLTVDKSR WQQGNVFSCSVMHEALHNHYTQKSLSLSPGK |

**Table 1-8. Sequences and sequence numbers corresponding to the sequences of anti-B7-H4 antibody PR003366**

| LCDR1 | LCDR2 | LCDR3 | HCDR1 | HCDR2 | HCDR3 |
|---|---|---|---|---|---|
| 78 | 80 | 82 | 72 | 74 | 76 |
| RASQSVSSNLA | GASTRAT | QQYKNWPFT | EDTFSSY | APIFGT | GGPYFDY |
| HC | | VL | | VH | |
| 87 | | 85 | | 84 | |
| EIVMTQSPASLSVSPGERATLSCRAS QSVSSNLAWYQQKPGQAPRLLIYGA STRATGIPARVSGGGSGTEFTLTISSL QSEDFAVYYCQQYKNWPFTFGPGTK LEIKGGGGSGGGGSGGGGSGGGGSQ VQLVQSGAEVKKPGSSMRVSCKASE DTFSSYAISWVRQAPGQGLEWMGGT APIFGTTNYAQKFQGRVTITADKSTS TAYMELSSLRSEDTAVYYCARGGPY FDYWGQGTLVTVSSGGGASEPKSCD KTHTCPPCPAPELLGGPSVFLFPPKPK DTLMISRTPEVTCVVVDVSHEDPEV KFNWYVDGVEVHNAKTKPREEQYN STYRVVSVLTVLHQDWLNGKEYKC KVSNKALPAPIEKTISKAKGQPREPQ VYTLPPSREEMTKNQVSLTCLVKGF YPSDIAVEWESNGQPENNYKTTPPVL DSDGSFFLYSKLTVDKSRWQQGNVF SCSVMHEALHNHYTQKSLSLSPGK | | EIVMTQSPASLSVSPGERA TLSCRASQSVSSNLAWYQ QKPGQAPRLLIYGASTRAT GIPARVSGGGSGTEFTLTIS SLQSEDFAVYYCQQYKNW PFTFGPGTKLEIK | | QVQLVQSGAEVKKPG SSMRVSCKASEDTFSS YAISWVRQAPGQGLE WMGGTAPIFGTTNYA QKFQGRVTITADKSTS TAYMELSSLRSEDTAV YYCARGGPYFDYWG QGTLVTVSS | |

### Example 2. Binding Ability of Anti-Human B7-H4 HCAb Monoclonal Antibodies at Cellular Level as Assayed by FACS

In this example, to investigate the *in vitro* binding activity of anti-human B7-H4 HCAb monoclonal antibodies for human B7-H4, antibody binding experiments at the cellular level were conducted using a CHO-K1 cell strain (CHO-K1/huB7-H4, Harbour BioMed) and a HEK293 cell strain (HEK293/huB7-H4, Harbour BioMed) overexpressing human B7-H4, a CHO-K1 cell strain overexpressing cynomolgus monkey B7-H4 (CHO-K1/cynoB7-H4, Harbour BioMed), a CHO-K1 cell strain overexpressing mouse B7-H4 (CHO-K1/mB7-H4, Harbour BioMed), and MDA-MB-468 breast cancer cells endogenously and highly expressing B7-H4. Briefly, cells were digested and resuspended in PBS containing 2% FBS, and the cell density was adjusted to 1 × 10⁶ cells/mL. The cells were seeded in a 96-well V-bottom plate (Corning, Cat# 3894) at 100 µL/well, followed by the addition of test antibodies 3-fold serially diluted at a concentration that was 2 times the final concentration, each at 100 µL/well. The cells were incubated away from light at 4 °C for 2 h. Then, the cells in each well were rinsed twice with 100 µL of pre-cooled PBS and centrifuged at 500 g at 4 °C for 5 min, and then the supernatant was discarded. Then, a fluorescent secondary antibody (Alexa Fluor 488-conjugated AffiniPure Goat Anti-Human IgG, Fcy Fragment Specific, Jackson, Cat#109-545-06, diluted in a 1:500 ratio) was added at 100 µL per well, and the cells were incubated away from light at 4 °C for 60 min. The cells in each well were then rinsed twice with 100 µL of pre-cooled PBS and centrifuged at 500 g at 4 °C for 5 min, and then the supernatant was discarded. Finally, the cells in each well were resuspended in 200 µL of pre-cooled PBS, and the fluorescence signal values were read using ACEA Novocyte3000.

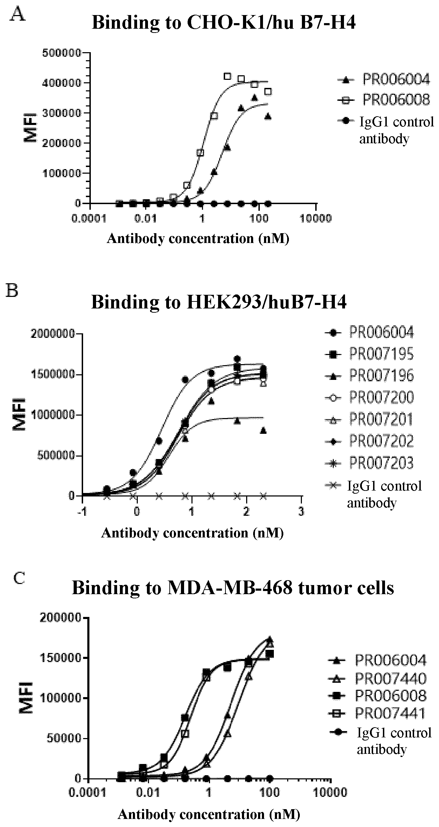

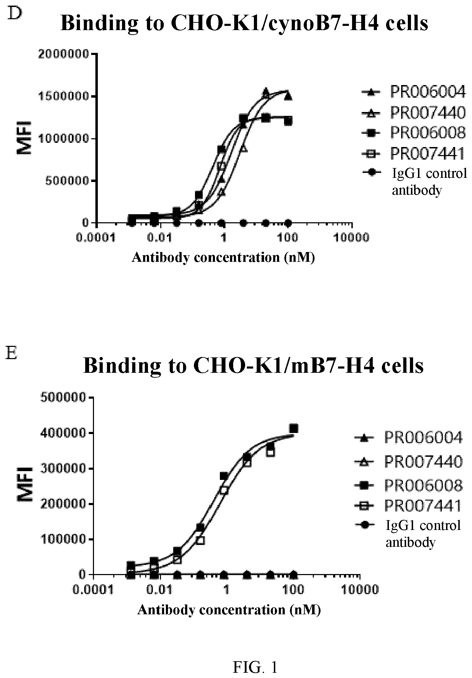

The results of the binding of antibodies to human B7-H4 on the cell surface are shown in Tables 2-1, 2-2, and 2-3, and FIGs. 1A-1E. The results show that PR006004 and PR006008 exhibited good binding to human and cynomolgus monkey B7-H4 and MDA-MB-468 tumor cells, wherein PR006008 had stronger cell binding and had cross reaction with mouse B7-H4. Binding of the mutant antibodies obtained by lowering isoelectric point of PR006004 to human B7-H4 was slightly reduced compared with that of PR006004 itself, with PR007196 having the weakest binding ability. The binding activity of the mutants PR007440 and PR007441 obtained by mutation of post-translational modification site (PTM) of PR006004 and PR006008 was not significantly changed compared with that of the parent antibodies.

**Table 2-1**

| | **CHOK1/hu B7-H4** | |
|---|---|---|
| **Antibody** | **Maximum MFI** | **EC50 (nM)** |
| PR006004 | 333458 | 4.863 |
| PR006008 | 404722 | 1.072 |

**Table 2-2**

| | **CHOK1/hu B7-H4** | |
|---|---|---|
| **Antibody** | **Maximum MFI** | **EC50 (nM)** |
| PR006004 | 1632510 | 2.755 |
| PR007195 | 1584634 | 5.65 |
| PR007196 | 971497 | 3.717 |
| PR007200 | 1477298 | 5.393 |
| PR007201 | 1460146 | 5.496 |
| PR007202 | 1508724 | 5.582 |
| PR007203 | 1527268 | 5.405 |

**Table 2-3**

| | MDA-MB-468 | | CHO-K1/cyno B7-H4 | | CHO-K1/m B7-H4 | |
|---|---|---|---|---|---|---|
| Antibody | Maximum **MFI** | EC50 (nM) | Maximum **MFI** | EC50 (nM) | Maximum **MFI** | EC50 (nM) |
| PR006004 | 182805 | 5.186 | 1573933 | 1.651 | ∼ | ∼ |
| PR007440 | 184529 | 9.178 | 1588512 | 3.056 | ∼ | ∼ |
| PR006008 | 149316 | 0.1616 | 1250247 | 0.432 | 399485 | 0.4026 |
| PR007441 | 148378 | 0.2439 | 1260379 | 0.7209 | 400110 | 0.6168 |

### Example 3. Determination of Affinity by BLI Method

10× kinetics buffer (ForteBio, #18-1105) was diluted to 1× kinetics buffer for affinity assay and dilution of antigens and antibodies. The binding kinetics between the antigen and the antibody was analyzed by the biolayer interferometry (BLI) technique using an Octet Red 96e molecular interaction analyzer (Fortebio).

When the affinity of the antigen for the antibody was determined, the rotation speed of the sensor was set at 1000 rpm/min. The AHC sensors (Fortebio, #18-5060) placed in a row were equilibrated for 10 min in a test buffer, and then the AHC sensors were used to capture the B7-H4 antibodies at a capture height of 0.7 nm; the AHC sensors, after equilibrated in the buffer for 120 s, bound to 2-fold serially diluted human or monkey B7-H4 (concentrations were 100 nM-3.75 nM and 0 nM) for 180 s, followed by dissociation for 300 s. Finally, the AHC sensor was immersed in a 10 mM glycine-hydrochloric acid solution at pH 1.5 for regeneration to elute the proteins bound to the sensor.

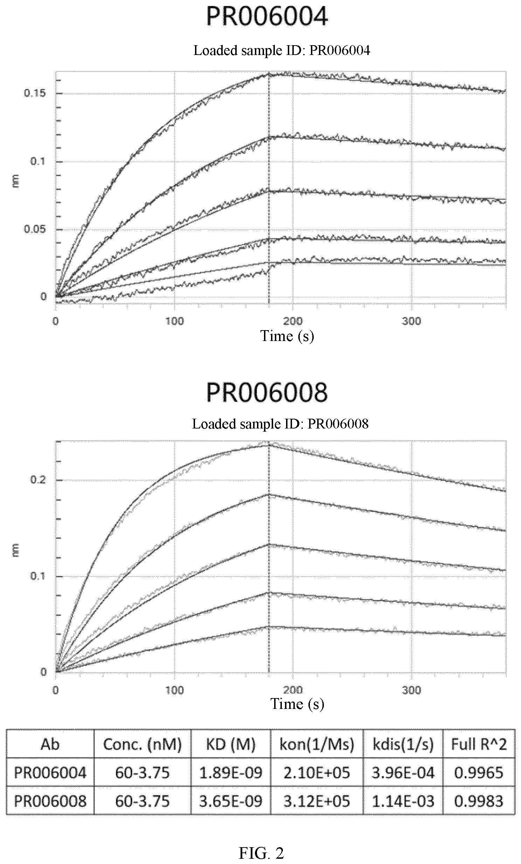

When data analysis was performed using Octet Data Analysis software (Fortebio, version 11.0), 0 nM was taken as a reference well, and reference subtraction was performed; the "1:1 Global fitting" method was selected to fit the data, and the kinetics parameters of the binding of antigens to antigen-binding proteins were calculated, with kₒₙ(1/Ms) values, k_{dis}(1/s) values, and K_{D}(M) values obtained (see Table 3 and FIG. 2). The results show that PR006004 and PR006008 both had good affinity at protein level, with the affinity of PR006004 slightly higher than that of PR006008. After the increase of the TM value of antibodies through amino acid mutations, PR007077 and PR007078 showed no significant change in affinity for B7-H4 compared with their corresponding parent antibodies PR006391 and PR006407.

**Table. 3**

| | Ab | Conc. (nM) | KD (M) | Kon (1/Ms) | Kdis (1/s) | Full R^2 |
|---|---|---|---|---|---|---|
| human B7-H4 | PR006004 | 60-3.75 | 1.89E-09 | 2.10E+05 | 3.96E-04 | 0.9965 |
| | PR006008 | 60-3.75 | 3.65E-09 | 3.12E+05 | 1.14E-03 | 0.9983 |
| | PR006391 | 100-6.25 | 3.75E-09 | 9.04E+04 | 3.39E-04 | 0.9983 |
| | PR006407 | 100-6.25 | 8.40E-09 | 1.50E+05 | 1.26E-03 | 0.999 |
| | PR007077 | 100-6.25 | 5.43E-09 | 1.09E+05 | 5.94E-04 | 0.9752 |
| | PR007078 | 100-6.25 | 1.03E-08 | 1.49E+05 | 1.54E-03 | 0.9965 |
| | PR007168 | 100-6.25 | 1.69E-10 | 1.83E+05 | 3.09E-05 | 0.9974 |
| cyno B7-H4 | PR007077 | 100-6.25 | 4.74E-08 | 1.72E+05 | 8.16E-03 | 0.9821 |
| | PR007078 | 100-6.25 | 6.78E-09 | 2.19E+05 | 1.49E-03 | 0.9966 |
| | PR007168 | 60-3.75 | 3.01E-10 | 2.94E+05 | 8.87E-05 | 0.9972 |

### Example 4. Structure and Design of B7-H4×CD3 Bispecific Antibodies

A B7-H4×CD3 bispecific antibody can bind to two targets simultaneously, with one terminus being capable of recognizing B7-H4 specifically expressed on tumor cell surfaces and the other terminus being capable of binding to CD3 molecules on T cells. After binding to the surface of a tumor cell, the B7-H4×CD3 bispecific antibody molecule can recruit and activate T cells in the vicinity of the tumor cell, thereby killing the tumor cell.

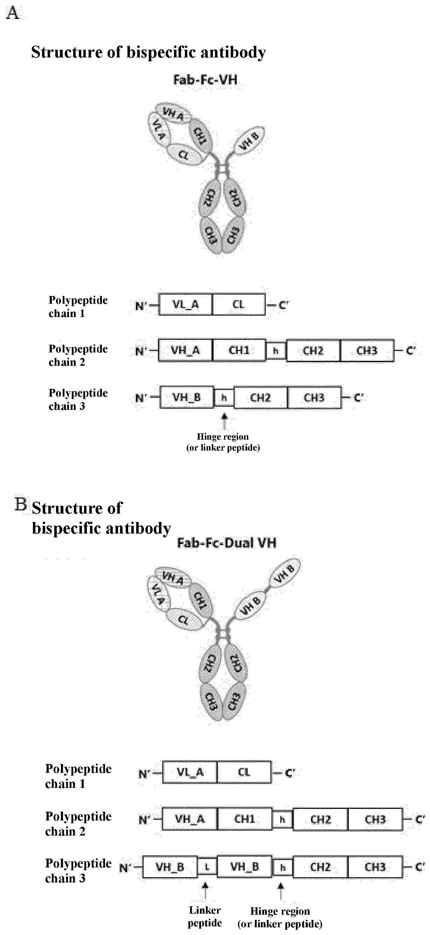

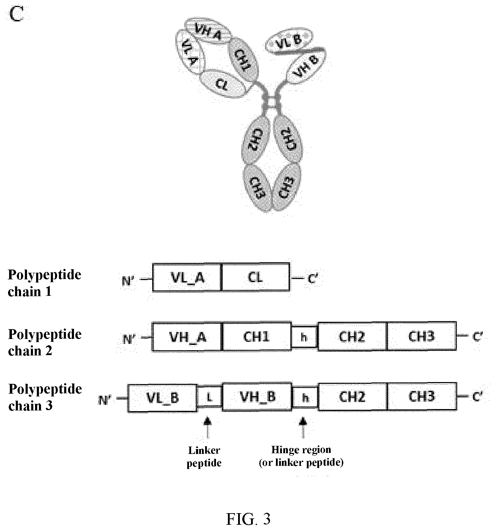

In this example, the anti-B7-H4 antibodies obtained in Example 1 and the anti-CD3 antibody PR003886 (from patent application WO2021/063330, sequences listed in Table 4-1) were used to prepare B7-H4×CD3 bispecific antibodies. The bispecific antibody has a structure shown in FIGs. 3A-3C. The structure comprises three polypeptide chains: a polypeptide chain 1 or a first polypeptide chain, comprising VL_A-CL from the amino-terminus to the carboxyl-terminus; a polypeptide chain-2 or a second polypeptide chain, comprising VH_A-CH1-h-CH2-CH3 from the amino-terminus to the carboxyl-terminus; and a polypeptide chain-3 or a third polypeptide chain, comprising VH_B-L-VH_B-h-CH2-CH3 or VH_B-h-CH2-CH3 from the amino-terminus to the carboxyl-terminus. VH_A and VL_A are heavy chain and light chain variable regions of an antibody A, respectively; VH_B is a heavy chain variable region of a heavy-chain antibody B; CL is a domain of a light chain constant region; CH1, CH2, and CH3 are first, second, and third domains of a heavy chain constant region, respectively; L is a linker peptide, and h is a hinge region or a derivative sequence of an IgG antibody.

The B7-H4×CD3 bispecific antibody molecules RP007354 and PR007355 in Table 4-2 have the same molecular structure, with the polypeptide chain 1 and the polypeptide chain-2 constituting the Fab portion targeting CD3 and the polypeptide chain-3 comprising a VH portion targeting B7-H4 (VH_B).

The other B7-H4×CD3 bispecific antibody molecules in Table 4-2 (PR006391, PR006407, PR006840, PR007077, PR007078, and PR007168) all have the same molecular structure, with the polypeptide chain 1 and the polypeptide chain-2 constituting the Fab portion targeting CD3 and the polypeptide chain-3 comprising two VH portions targeting B7-H4 (VH_B). The sequences of the two VH_B may be identical or different, and the two VH_B are linked via a linker peptide GS_15 (SEQ ID NO: 66). In addition, PR005885 is a "1+1" Fab-Fc-scFv asymmetric structural molecule, the structure of which involves three protein chains, which comprise the scFv polypeptide chains of the corresponding anti-B7-H4 antibody, and the heavy and light chains of the above anti-CD3 antibody, respectively.

To prevent crosslinking and reduced effector functions caused by Fcy receptor binding, "AA" double mutations (L234A and L235A) or "AAA" triple mutants (L234A, L235A, and G237A) were introduced into the heavy chain constant regions of the polypeptide chain-2 and the polypeptide chain-3. Furthermore, to reduce the production of heavy chain homodimers, different amino acid mutations were introduced into the constant regions of the two heavy chains, such that they could carry a "knob-hole" mutation and a modified disulfide bond. Mutations T366W and S354C were introduced into the polypeptide chain-2 targeting CD3; meanwhile, mutations T366S, L368A, Y407V, and Y349C were introduced into the polypeptide chain-3 targeting B7-H4. The Fc constant region sequence of polypeptide chain-2 is set forth in SEQ ID NO: 68, and the Fc constant region sequence of polypeptide chain-3 is set forth in SEQ ID NO: 67. Further, to improve the thermostability of the antibody molecule, an additional disulfide bond was introduced into the VH domain of the anti-B7-H4 antibody according to patent WO2012100343A1; specifically, two amino acids at positions 49 and 69 (according to the Kabat numbering scheme) in the VH domain of the anti-B7-H4 antibody into Cys to allow them to form a disulfide bond; namely, mutations S49C and I70C were introduced, respectively. The B7-H4×CD3 bispecific antibody molecules and their parent monoclonal antibodies at the CD3 end and their parent monoclonal antibodies at the B7-H4 end were listed in Table 4-2; the corresponding sequence numbers of the polypeptide chain sequences of the B7-H4×CD3 bispecific antibodies of the present invention are listed in Table 4-3.

The expression vectors encoding three polypeptide chains were each co-transfected into mammalian host cells for recombinant expression using the method described in Example 1.5, and the purified antibody protein molecules were obtained.

The data about stability Tm, solubility HIC, analysis of byproducts after one-step purification, etc. are shown in Table 1-3.

**Table 4-1. Sequence numbers corresponding to the sequences of the anti-CD3 antibody PR003886 used in the present invention**

| **VL** | **VH** | **LCDR1** | **LCDR2** | **LCDR3** | **HCDR1** | **HCDR2** | **HCDR3** |
|---|---|---|---|---|---|---|---|
| 46 | 35 | 29 | 31 | 33 | 3 | 10 | 23 |
| QAVVTQEPSLT VSPGGTVTLTC RSSTGAVTTSN YANWVQQKP GQAPRGLIGG TNKRAPWTPA RFSGSLLGDK AALTLLGAQP EDEAEYFCAL WYSNLWVFG GGTKLTVL | EVQLVESGGGLV QPGGSLKLSCAA SGFTFSTYAMN WVRQASGKGLE WVGRIRSKYNN YATYYADSVKDR FTISRDDSKNTA YLQMNSLKTED TAVYYCTRHGNF GNSYVSWFAYW GQGTLVTVS S | RSSTGAV TTSNYAN | GTNKRAP | ALWYSN LWV | GFTFSTY | | RSKYN HGNFG NYA NSYVS WFAY |

| **Light chain** | | **Heavy chain** | | | | | |
|---|---|---|---|---|---|---|---|
| 58 | | 47 | | | | | |
| QAVVTQEPSLTVSPGGTVTLTCRS STGAVTTSNYANWVQQKPGQAPR GLIGGTNKRAPWTPARFSGSLLGD KAALTLLGAQPEDEAEYFCALWY SNLWVFGGGTKLTVLGQPKAAPS VTLFPPSSEELQANKATLVCLISDF YPGAVTVAWKADSSPVKAGVETT TPSKQSNNKYAASSYLSLTPEQWK SHRSYSCQVTHEGSTVEKTVAPTE CS | | EVQLVESGGGLVQPGGSLKLSCAASGFTFSTYAMNWVRQASGKG LEWVGRIRSKYNNYATYYADSVKDRFTISRDDSKNTAYLQMNSLK TEDTAVYYCTRHGNFGNSYVSWFAYWGQGTLVTVSSASTKGPSVF PLAPSSKSTSGGTAALGCLVKDYFPEPVTVSWNSGALTSGVHTFPA VLQSSGLYSLSSVVTVPSSSLGTQTYICNVNHKPSNTKVDKKVEPK SCDKTHTCPPCPAPEAAGGPSVFLFPPKPKDTLMISRTPEVTCVVV DVSHEDPEVKFNWYVDGVEVHNAKTKPREEQYNSTYRVVSVLTV LHQDWLNGKEYKCKVSNKALPAPIEKTISKAKGQPREPQVYTLPP SREEMTKNQVSLTCLVKGFYPSDIAVEWESNGQPENNYKTTPPVL DSDGSFFLYSKLTVDKSRWQQGNVFSCSVMHEALHNHYTQKSLSL SPGK | | | | | |

**Table 4-2. B7-H4×CD3 bispecific antibody molecules and parent monoclonal antibodies thereof**

| **Bispecific antibody molecules** | **CD3 antibody** | **B7-H4 antibody** |
|---|---|---|
| PR007354 | PR003886 | Monovalent PR007440 |
| PR007355 | PR003886 | Monovalent PR007441 |
| PR006391 | PR003886 | Bivalent PR007440 |
| PR006407 | PR003886 | Bivalent PR007441 |
| PR006840 | PR003886 | PR007440 and PR007441 |
| PR007077 | PR003886 | Bivalent PR007440, addition (S49C, 170C) |
| PR007078 | PR003886 | Bivalent PR007441, addition (S49C, 170C) |
| PR007168 | PR003886 | PR007440 and PR007441, each with addition (S49C, I70C) |
| PR005885 | PR003886 | PR003366 |

**Table 4-3. Corresponding sequence numbers of the polypeptide chain sequences of the B7-H4×CD3 bispecific antibodies of the present invention**

| **Antibody No.** | **Polypeptide chain 1** | | **Pol ypeptide chain-2** | | | **Polypeptide chain-3** |
|---|---|---|---|---|---|---|
| PR006391 | 58 | QAVV TQEPS LTVSP GGTV TLTCR SSTG AVTTS NYAN WVQQ KPGQ APRG LIGGT NKRA PWTP ARFS GSLL GDKA ALTLL GAQP EDEA EYFC ALWY SNLW VFGG GTKL TVLG QPKA APSV TLFPP SSEEL QANK ATLVC LISDF YPGA VTVA WKAD SSPVK AGVE TTTPS KQSN NKYA ASSY | 59 | EVQLVE SGGGLV QPGGSL KLSCAA SGFTFST YAMNW VRQASG KGLEW VGRIRS KYNNYA TYYADS VKDRFT ISRDDSK NTAYLQ MNSLKT EDTAVY YCTRHG NFGNSY VSWFAY WGQGT LVTVSS ASTKGP SVFPLAP SSKSTSG GTAALG CLVKDY FPEPVT VSWNSG ALTSGV HTFPAVL QSSGLY SLSSVV TVPSSSL GTQTYI CNVNHK PSNTKV DKKVEP KSCDKT HTCPPC PAPEAA GAPSVF LFPPKPK | 60 | EVQLVESGGGLVQPGGSLRLSCAASGFAFSNYW MHWARQVPGKGRVWISRISGDSRSTSYADSVKG RFTISRDNAKNMVYLQMNNLRAEDTAVYYCAR DRFGDDYYYGMNVWGQGTTVTVSSGGGGSGG GGSGGGGSEVQLVESGGGLVQPGGSLRLSCAAS GFAFSNYWMHWARQVPGKGRVWISRISGDSRST SYADSVKGRFTISRDNAKNMVYLQMNNLRAED TAVYYCARDRFGDDYYYGMNVWGQGTTVTVSS ASEPKSSDKTHTCPPCPAPEAAGAPSVFLFPPKPK DTLMISRTPEVTCVVVDVSHEDPEVKFNWYVDG VEVHNAKTKPREEQYNSTYRVVSVLTVLHQDW LNGKEYKCKVSNKALPAPIEKTISKAKGQPREPQ VCTLPPSREEMTKNQVSLSCAVKGFYPSDIAVEW ESNGQPENNYKTTPPVLDSDGSFFLVSKLTVDKS RWQQGNVFSCSVMHEALHNHYTQKSLSLSPGK |
| PR006407 | 58 | | 59 | | 61 | EVQLVESGGGLVQPGGSLRLSCAASGFTFTDFW MHWVRQVPGKGREWVSRISPDSSSTSYEDSVKG RFTISRDNAKNTVYLQMHGLRAEDTAVYYCTRF STGWHRIEYFQHWGQGTLVTVSSGGGGSGGGGS GGGGSEVQLVESGGGLVQPGGSLRLSCAASGFTF TDFWMHWVRQVPGKGREWVSRISPDSSSTSYED SVKGRFTISRDNAKNTVYLQMHGLRAEDTAVYY CTRFSTGWHRIEYFQHWGQGTLVTVSSASEPKSS DKTHTCPPCPAPEAAGAPSVFLFPPKPKDTLMISR TPEVTCVVVDVSHEDPEVKFNWYVDGVEVHNA KTKPREEQYNSTYRVVSVLTVLHQDWLNGKEY KCKVSNKALPAPIEKTISKAKGQPREPQVCTLPPS REEMTKNQVSLSCAVKGFYPSDIAVEWESNGQP ENNYKTTPPVLDSDGSFFLVSKLTVDKSRWQQG NVFSCSVMHEALHNHYTQKSLSLSPGK |
| PR006840 | 58 | | 59 | | 62 | EVQLVESGGGLVQPGGSLRLSCAASGFAFSNYW MHWARQVPGKGRVWISRISGDSRSTSYADSVKG RFTISRDNAKNMVYLQMNNLRAEDTAVYYCAR DRFGDDYYYGMNVWGQGTTVTVSSGGGGSGG GGSGGGGSEVQLVESGGGLVQPGGSLRLSCAAS GFTFTDFWMHWVRQVPGKGREWVSRISPDSSST SYEDSVKGRFTISRDNAKNTVYLQMHGLRAEDT AVYYCTRFSTGWHRIEYFQHWGQGTLVTVSSAS EPKSSDKTHTCPPCPAPEAAGAPSVFLFPPKPKDT LMISRTPEVTCVVVDVSHEDPEVKFNWYVDGVE VHNAKTKPREEQYNSTYRVVSVLTVLHQDWLN GKEYKCKVSNKALPAPIEKTISKAKGQPREPQVC |
| | | LSLTP EQWK SHRS YSCQ VTHE GSTV EKTV APTE CS | | DTLMIS RTPEVT CVVVDV SHEDPE VKFNW YVDGVE VHNAKT KPREEQ YNSTYR VVSVLT VLHQD WLNGK EYKCKV SNKALP APIEKTI SKAKGQ PREPQV YTLPPC REEMTK NQVSLW CLVKGF YPSDIAV EWESNG QPENNY KTTPPV LDSDGS FFLYSKL TVDKSR WQQGN VFSCSV MHEALH NHYTQK SLSLSPG K | | TLPPSREEMTKNQVSLSCAVKGFYPSDIAVEWES NGQPENNYKTTPPVLDSDGSFFLVSKLTVDKSRW QQGNVFSCSVMHEALHNHYTQKSLSLSPGK |
| PR007077 | 58 | | 59 | | 63 | EVQLVESGGGLVQPGGSLRLSCAASGFAFSNYW MHWARQVPGKGRVWICRISGDSRSTSYADSVKG RFTCSRDNAKNMVYLQMNNLRAEDTAVYYCAR DRFGDDYYYGMNVWGQGTTVTVSSGGGGSGG GGSGGGGSEVQLVESGGGLVQPGGSLRLSCAAS GFAFSNYWMHWARQVPGKGRVWICRISGDSRST SYADSVKGRFTCSRDNAKNMVYLQMNNLRAED TAVYYCARDRFGDDYYYGMNVWGQGTTVTVSS ASEPKSSDKTHTCPPCPAPEAAGAPSVFLFPPKPK DTLMISRTPEVTCVVVDVSHEDPEVKFNWYVDG VEVHNAKTKPREEQYNSTYRVVSVLTVLHQDW LNGKEYKCKVSNKALPAPIEKTISKAKGQPREPQ VCTLPPSREEMTKNQVSLSCAVKGFYPSDIAVEW ESNGQPENNYKTTPPVLDSDGSFFLVSKLTVDKS RWQQGNVFSCSVMHEALHNHYTQKSLSLSPGK |
| PR007078 | 58 | | 59 | | 64 | EVQLVESGGGLVQPGGSLRLSCAASGFTFTDFW MHWVRQVPGKGREWVCRISPDSSSTSYEDSVKG RFTCSRDNAKNTVYLQMHGLRAEDTAVYYCTRF STGWHRIEYFQHWGQGTLVTVSSGGGGSGGGGS GGGGSEVQLVESGGGLVQPGGSLRLSCAASGFTF TDFWMHWVRQVPGKGREWVCRISPDSSSTSYE DSVKGRFTCSRDNAKNTVYLQMHGLRAEDTAV YYCTRFSTGWHRIEYFQHWGQGTLVTVSSASEP KSSDKTHTCPPCPAPEAAGAPSVFLFPPKPKDTL MISRTPEVTCVVVDVSHEDPEVKFNWYVDGVEV HNAKTKPREEQYNSTYRVVSVLTVLHQDWLNG KEYKCKVSNKALPAPIEKTISKAKGQPREPQVCT LPPSREEMTKNQVSLSCAVKGFYPSDIAVEWESN GQPENNYKTTPPVLDSDGSFFLVSKLTVDKSRWQ QGNVFSCSVMHEALHNHYTQKSLSLSPGK |
| PR007168 | 58 | | 59 | | 65 | EVQLVESGGGLVQPGGSLRLSCAASGFAFSNYW MHWARQVPGKGRVWICRISGDSRSTSYADSVKG RFTCSRDNAKNMVYLQMNNLRAEDTAVYYCAR DRFGDDYYYGMNVWGQGTTVTVSSGGGGSGG GGSGGGGSEVQLVESGGGLVQPGGSLRLSCAAS GFTFTDFWMHWVRQVPGKGREWVCRISPDSSST SYEDSVKGRFTCSRDNAKNTVYLQMHGLRAED TAVYYCTRFSTGWHRIEYFQHWGQGTLVTVSSA SEPKSSDKTHTCPPCPAPEAAGAPSVFLFPPKPKD TLMISRTPEVTCVVVDVSHEDPEVKFNWYVDGV EVHNAKTKPREEQYNSTYRVVSVLTVLHQDWL NGKEYKCKVSNKALPAPIEKTISKAKGQPREPQV CTLPPSREEMTKNQVSLSCAVKGFYPSDIAVEWE SNGQPENNYKTTPPVLDSDGSFFLVSKLTVDKSR WQQGNVFSCSVMHEALHNHYTQKSLSLSPGK |
| PR007354 | 58 | | 59 | | 69 | EVQLVESGGGLVQPGGSLRLSCAASGFAFSNYW MHWARQVPGKGRVWICRISGDSRSTSYADSVKG RFTCSRDNAKNMVYLQMNNLRAEDTAVYYCAR DRFGDDYYYGMNVWGQGTTVTVSSASEPKSSD |
| | | | | | | KTHTCPPCPAPEAAGAPSVFLFPPKPKDTLMISRT PEVTCVVVDVSHEDPEVKFNWYVDGVEVHNAK TKPREEQYNSTYRVVSVLTVLHQDWLNGKEYKC KVSNKALPAPIEKTISKAKGQPREPQVCTLPPSRE EMTKNQVSLSCAVKGFYPSDIAVEWESNGQPEN NYKTTPPVLDSDGSFFLVSKLTVDKSRWQQGNV FSCSVMHEALHNHYTQKSLSLSPGK |
| PR007355 | 58 | | 59 | | 70 | EVQLVESGGGLVQPGGSLRLSCAASGFTFTDFW MHWVRQVPGKGREWVCRISPDSSSTSYEDSVKG RFTCSRDNAKNTVYLQMHGLRAEDTAVYYCTRF STGWHRIEYFQHWGQGTLVTVSSASEPKSSDKT HTCPPCPAPEAAGAPSVFLFPPKPKDTLMISRTPE VTCVVVDVSHEDPEVKFNWYVDGVEVHNAKTK PREEQYNSTYRVVSVLTVLHQDWLNGKEYKCK VSNKALPAPIEKTISKAKGQPREPQVCTLPPSREE MTKNQVSLSCAVKGFYPSDIAVEWESNGQPENN YKTTPPVLDSDGSFFLVSKLTVDKSRWQQGNVFS CSVMHEALHNHYTQKSLSLSPGK |
| PR005885 | 58 | | 59 | | 86 | EIVMTQSPASLSVSPGERATLSCRASQSVSSNLAW YQQKPGQAPRLLIYGASTRATGIPARVSGGGSGT EFTLTISSLQSEDFAVYYCQQYKNWPFTFGPGTK LEIKGGGGSGGGGSGGGGSGGGGSQVQLVQSGA EVKKPGSSMRVSCKASEDTFSSYAISWVRQAPGQ GLEWMGGTAPIFGTTNYAQKFQGRVTITADKSTS TAYMELSSLRSEDTAVYYCARGGPYFDYWGQGT LVTVSSASEPKSSDKTHTCPPCPAPEAAGAPSVFL FPPKPKDTLMISRTPEVTCVVVDVSHEDPEVKFN WYVDGVEVHNAKTKPREEQYNSTYRVVSVLTV LHQDWLNGKEYKCKVSNKALPAPIEKTISKAKG QPREPQVCTLPPSREEMTKNQVSLSCAVKGFYPS DIAVEWESNGQPENNYKTTPPVLDSDGSFFLVSK LTVDKSRWQQGNVFSCSVMHEALHNHYTQKSL SLSPGK |

### Example 5. In Vitro Binding of B7-H4×CD3 Bispecific Antibodies on CHO-K1 Cell Strain Overexpressing Human, Cynomolgus Monkey, and Mouse B7-H4 as Assayed by FACS

This example is intended to investigate the *in vitro* binding activity of the CD3 bispecific antibodies of B7-H4 for human, cynomolgus monkey, and mouse B7-H4. Antibody binding experiments at the cellular level were conducted using a CHO-K1 cell strain overexpressing human B7-H4 (CHO-K1/huB7-H4, Harbour BioMed), a CHO-K1 cell strain overexpressing cynomolgus monkey B7-H4 (CHO-K1/cynoB7-H4, Harbour BioMed), and a CHO-K1 cell strain overexpressing mouse B7-H4 (CHO-K1/mB7-H4, Harbour BioMed). Briefly, cells were digested and resuspended in PBS containing 2% FBS, and the cell density was adjusted to 1 × 10⁶ cells/mL. The cells were seeded in a 96-well V-bottom plate (Corning, Cat# 3894) at 100 µL/well, followed by the addition of test antibodies 3-fold serially diluted at a concentration that was 2 times the final concentration, each at 100 µL/well. The cells were incubated away from light at 4 °C for 2 h. Then, the cells in each well were rinsed twice with 100 µL of pre-cooled PBS and centrifuged at 500 g at 4 °C for 5 min, and then the supernatant was discarded. Then, a fluorescent secondary antibody (Alexa Fluor 488-conjugated AffiniPure Goat Anti-Human IgG, Fcy Fragment Specific, Jackson, Cat#109-545-06, diluted in a 1:500 ratio) was added at 100 µL per well, and the cells were incubated away from light at 4 °C for 60 min. The cells in each well were then rinsed twice with 100 µL of pre-cooled PBS and centrifuged at 500 g at 4 °C for 5 min, and then the supernatant was discarded. Finally, the cells in each well were resuspended in 200 µL of pre-cooled PBS, and the fluorescence signal values were read using ACEA Novocyte3000.

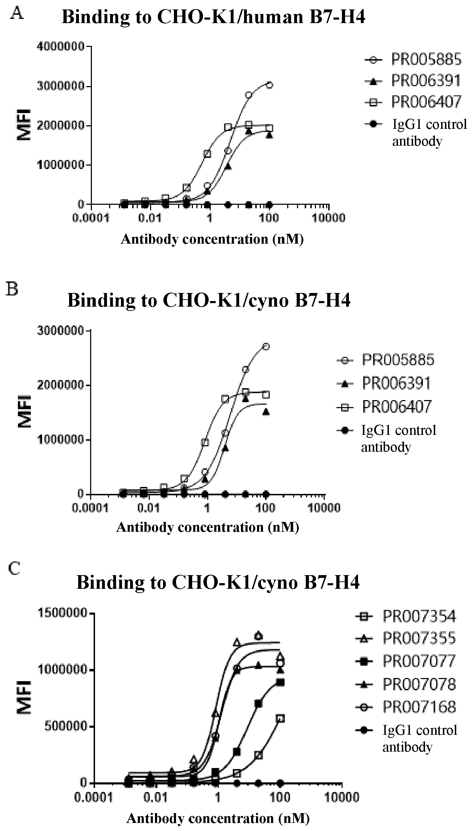

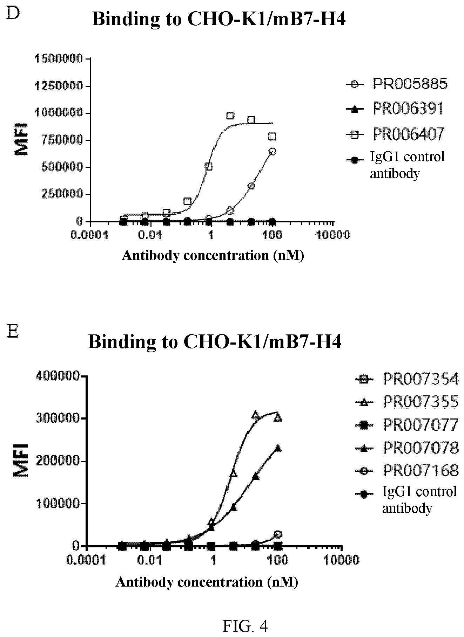

The results of the binding of antibodies to human B7-H4, cynomolgus monkey B7-H4, and mouse B7-H4 on the cell surface are shown in Table 5 and FIGs. 4A-4E. The results show that the bispecific antibody molecules PR006391, PR006407, and PR005885 exhibited good binding for both human and cynomolgus monkey B7-H4, wherein the bispecific antibody molecule PR006407 showed stronger binding for human and cynomolgus monkey B7-H4 compared with PR006391, the binding trend was consistent with the binding ability of their parent B7-H4 molecules PR006008 and PR006004, and PR005885 had a higher maximum MFI value. PR006407 had cross reaction with mouse B7-H4, PR006391 did not bind to mouse B7-H4, and PR005885 had weak cross reaction with mouse B7-H4. The binding trend of other antibodies PR007354, PR007355, PR007077, PR007078, and PR007168 was consistent with the binding ability of their parent B7-H4 molecules PR006008 and PR006004, wherein PR007355 and PR007078 had cross reaction with mouse B7-H4.

**Table. 5**

| | CHO-K1/hu B7-H4 | | CHO-K1/cyno B7-H4 | | CHO-K1/m B7-H4 | |
|---|---|---|---|---|---|---|
| Antibody | Maximum MFI | EC50 (nM) | Maximum MFI | EC50 (nM) | Maximum MFI | EC50 (nM) |
| PR006391 | 1892379 | 3.541 | 1668460 | 3.79 | ∼ | ∼ |
| PR006407 | 2017672 | 0.5232 | 1882190 | 0.7934 | 905150 | 0.7021 |
| PR007354 | | | 1381455 | 161.3 | ∼ | ∼ |
| PR007355 | | | 1241666 | 0.8321 | 319200 | 3.332 |
| PR007077 | | | 947650 | 8.587 | ∼ | ∼ |
| PR007078 | | | 1032094 | 1.044 | 301560 | 14.58 |
| PR007168 | | | 1180925 | 1.266 | ∼ | ∼ |
| PR005885 | 3173877 | 4.785 | 2889346 | 5.919 | 904379 | 37.00 |

### Example 6. In Vitro Binding of B7-H4×CD3 Bispecific Antibodies on Tumor Cells Endogenously Expressing B7-H4 and T Cells as Assayed by FACS

Antibody binding experiments at the cellular level were performed using tumor cells endogenously expressing B7-H4 or primary T cells expressing CD3. MDA-MB-468 cells or human T cells were resuspended in PBS containing 2% BSA. The cell density was adjusted to 1 × 10⁶ cells/mL. The cells were seeded in a 96-well V-bottom plate (Corning, # 3894) at 100 µL/well, followed by the addition of test antibodies 3-fold serially diluted at a concentration that was 2 times the final concentration, each at 100 µL/well. The cells were incubated away from light at 4 °C for 2 h. Then, the cells in each well were rinsed twice with 100 µL of pre-cooled PBS containing 2% BSA and centrifuged at 500 g at 4 °C for 5 min, and then the supernatant was discarded. Then, a fluorescent secondary antibody (Alexa Fluor 488-conjugated AffiniPure Goat Anti-Human IgG, Fcy Fragment Specific, Jackson, #109-545-098, diluted in a 1:500 ratio) was added at 100 µL per well, and the cells were incubated away from light at 4 °C for 1 h. The cells in each well were rinsed twice with 100 µL of pre-cooled PBS containing 2% BSA and centrifuged at 500 g at 4 °C for 5 min, and then the supernatant was discarded. Finally, the cells in each well were resuspended with 200 µL of pre-cooled PBS containing 2% BSA, and the fluorescence signal values were read using an ACEA Novocyte3000 flow cytometer.

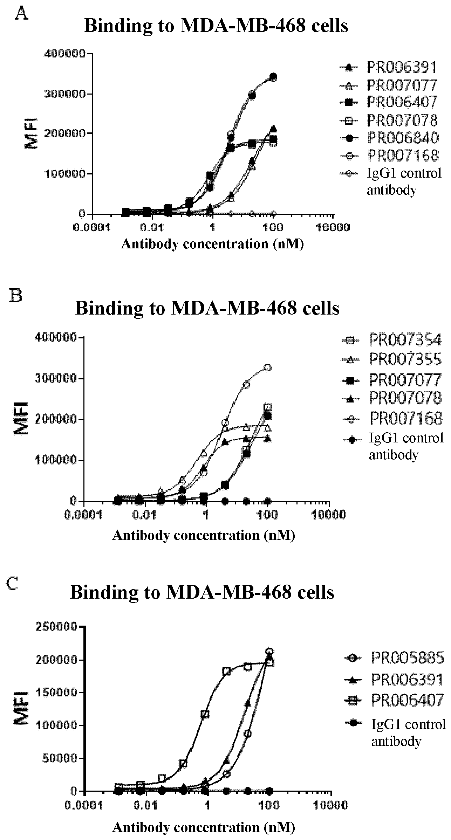

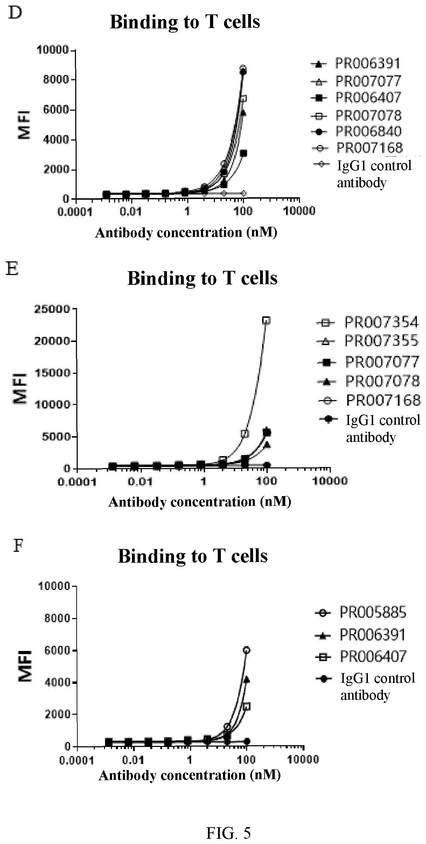

The results are shown in Tables 6-1, 6-2, and 6-3 and FIGs. 5A-5F. The results show that the bispecific antibody molecules all showed good binding ability for MDA-MB-468, wherein the bispecific antibody molecule PR006407 showed stronger binding for MDA-MB-468 than PR006391, and the results were consistent with the binding trend for CHO-K1/hu B7-H4; PR006840 had a higher maximum MFI. After the increase of the Tm value of antibodies through amino acid mutations, PR007077, PR007078, and PR007168 showed the same binding ability for MDA-MB-468 compared with their parent antibodies PR006391, PR006407, and PR006840. The monovalent bispecific antibody molecules RP007354 and PR007355 for B7-H4 showed no significant difference in binding compared with the bivalent bispecific antibody molecules PR007077 and PR007078 for B7-H4. The binding curve of PR005885 and MDA-MB-468 was similar to that of PR006391. Because an anti-CD3 antibody with weak affinity was adopted for the anti-CD3 end, the antibodies were all weak in binding to T cells, wherein PR007354 showed enhanced binding to the T cells compared with other antibodies.

**Table 6-1**

| | MDA-MB-468 | |
|---|---|---|
| Antibody | Maximum MFI | EC50 (nM) |
| PR006391 | 257406 | 19.13 |
| PR007077 | 279380 | 28.23 |
| PR006407 | 185290 | 0.7753 |
| PR007078 | 178616 | 0.9973 |
| PR006840 | 356834 | 3.758 |
| PR007168 | 346731 | 3.246 |

**Table 6-2**

| | MDA-MB-468 | |
|---|---|---|
| Antibody | Maximum MFI | EC50 (nM) |
| PR007354 | 305519 | 30.03 |
| PR007355 | 186462 | 0.4732 |
| PR007077 | 274432 | 28.83 |
| PR007078 | 158199 | 0.7463 |
| PR007168 | 336461 | 3.225 |

**Table 6-3**

| | MDA-MB-468 | |
|---|---|---|
| Antibody | Maximum MFI | EC50 (nM) |
| PR005885 | 412128 | 92.59 |
| PR006391 | 235559 | 15.75 |
| PR006407 | 195969 | 0.59 |

### Example 7. T Cell Killing Experiment

In this experiment, human primary T cells were used as effector cells, and MDA-MB-468 cells with high expression of B7-H4, OVCAR-3 cells with low expression of B7-H4, DLD-1 cells with extremely low expression of B7-H4, or completely negative cells MDA-MB-231 were used as target cells. The killing efficiency was reflected by the conductivity of the target cells measured using an RTCA instrument from ACEA. A 96-well plate e-plate was first equilibrated with 50 µL of complete medium. Target cells were digested, resuspended in RPM1640 complete medium containing 10% fetal bovine serum, and diluted to 4 × 10⁵/mL. The cell suspension was plated on the 96-well e-plate at 50 µL/well, i.e., 2 × 10⁴/well and incubated overnight at 37 °C. The next day, primary T cells were isolated using the T cell isolation kit (Miltenyi, #130-096-535) according to the method in the instructions. 50 µL of fresh culture medium containing 2 × 10⁵ T cells was added to each well, followed by the addition of 50 µL of antibody diluted in a 4× concentration gradient with a maximum final concentration of 10 nM. A total of 8 concentrations were set in duplicate for each antibody. The conductivity of the target cells was measured in real time. The value at 24 h was used to calculate the target cell killing efficiency according to the formula: target cell killing efficiency = (1 - sample/blank control) × 100%. The supernatant was collected after 24 h and the concentration of IFN-γ was detected by ELISA method. The instructions of IFN gamma Human Uncoated ELISA Kit (Thermo, #88-7316-77) were referred to for the ELISA method.

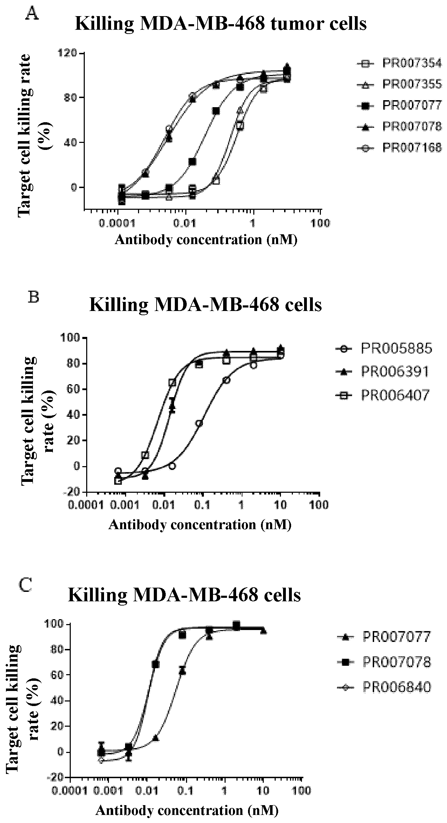

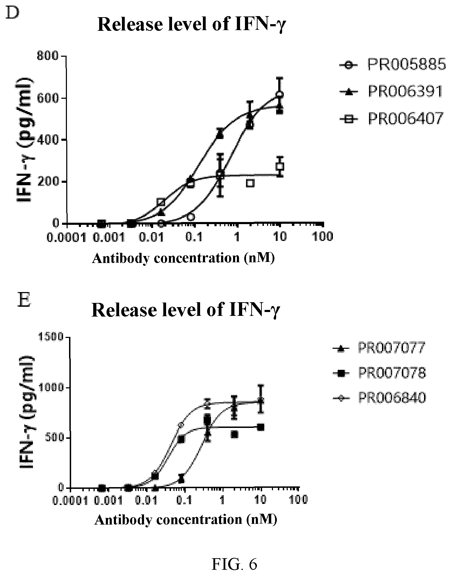

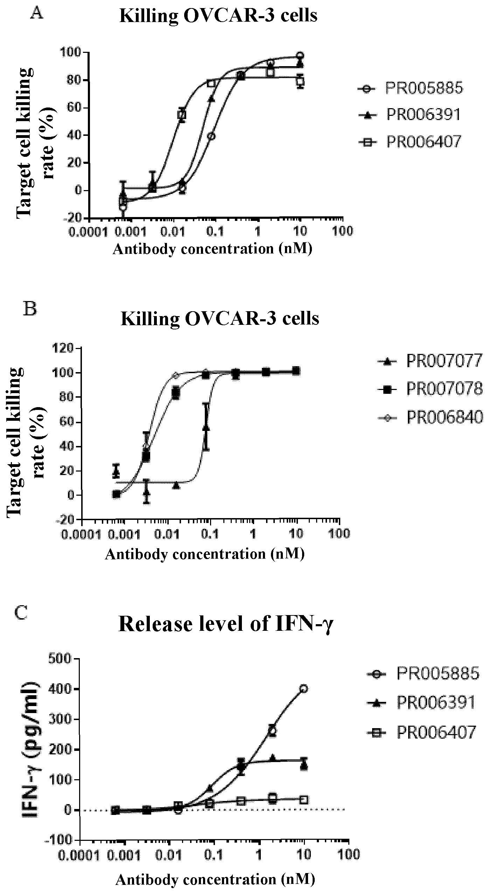

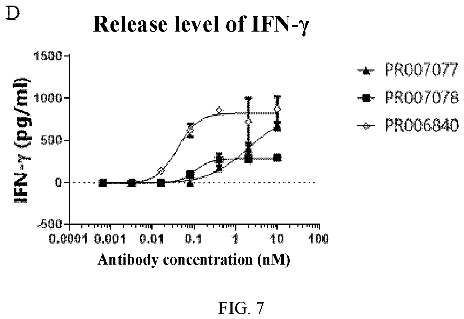

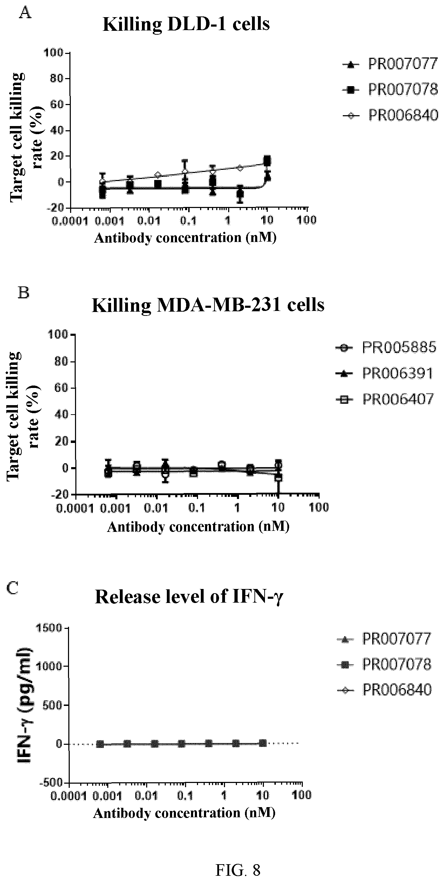

The results are shown in FIGs. 6A-6E, FIGs. 7A-7D, and FIGs. 8A-8C. The results show that all the bispecific antibody molecules exhibited significant killing effect on tumor cells expressing B7-H4. The bivalent bispecific antibody molecules PR007077 and PR007078 for B7-H4 showed significantly enhanced killing effect compared with the monovalent bispecific antibody molecules PR007354 and PR007355. Compared with PR006391, the bispecific antibody molecule PR006407 exhibited higher killing effect on MDA-MB-468 cells with high expression of B7-H4 or OVCAR-3 cells with medium expression of B7-H4 and exhibited lower expression of cytokine IFN-gamma; it showed no killing effect on MDA-MB-231 cells that did not express B7-H4. Similarly, the bispecific antibody molecule PR007078 with TM value increased through amino acid mutation exhibited higher killing effect on MDA-MB-468 and OVCAR-3 cells and lower expression of cytokine IFN-gamma compared with PR007077; it showed no killing effect on DLD-1 cells with extremely low expression of B7-H4. The bispecific antibody PR006840 exhibited both strong killing effect and higher cytokine secretion.

### Example 8. Experiment on Inducing Non-Specific Cytokines

In this experiment, the condition that the bispecific antibody molecules induced PBMCs to generate non-specific cytokines in the absence of target cells was detected, so that the safety of the drug could be preliminarily determined. Human PBMCs were resuspended in RPM1640 complete medium containing 10% fetal bovine serum and diluted to 2 × 10⁶/mL. The cell suspension was plated on a 96-well plate (Corning, 3599) at 100 µL/well, i.e., 2 × 10⁵/well, 100 µL of 2× antibody was added, and the mixture was incubated overnight at 37 °C. The supernatant was collected after 24 h, and the concentrations of IL-6, TNF-α, and IFN-y were detected by ELISA method. The ELISA detection method was performed according to the procedures in instructions of TNF-α human Uncoated ELISA Kit (Thermo, #88-7346-88), IL-6 human Uncoated ELISA Kit (Thermo, #88-7066-88), and IFN gamma Human Uncoated ELISA Kit (Thermo, #88-7316-77).

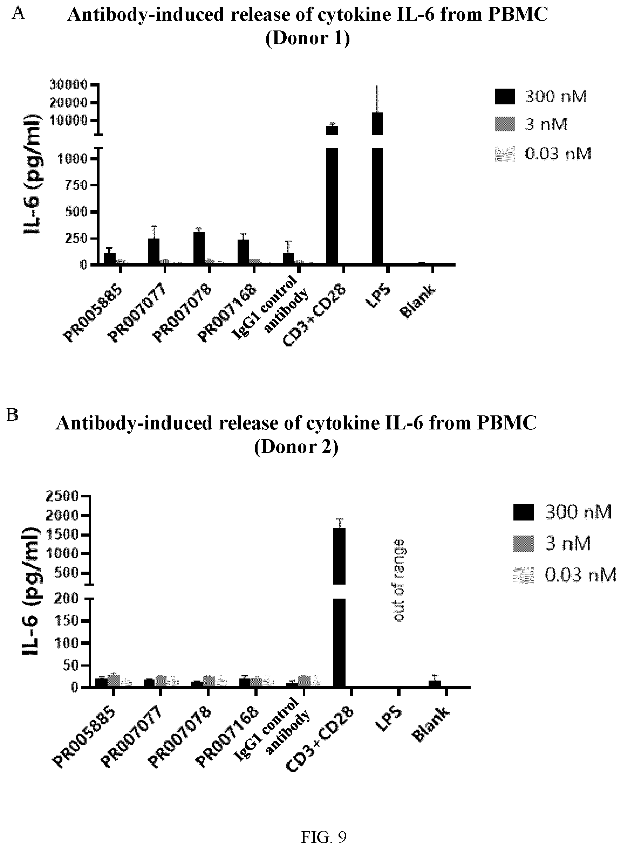

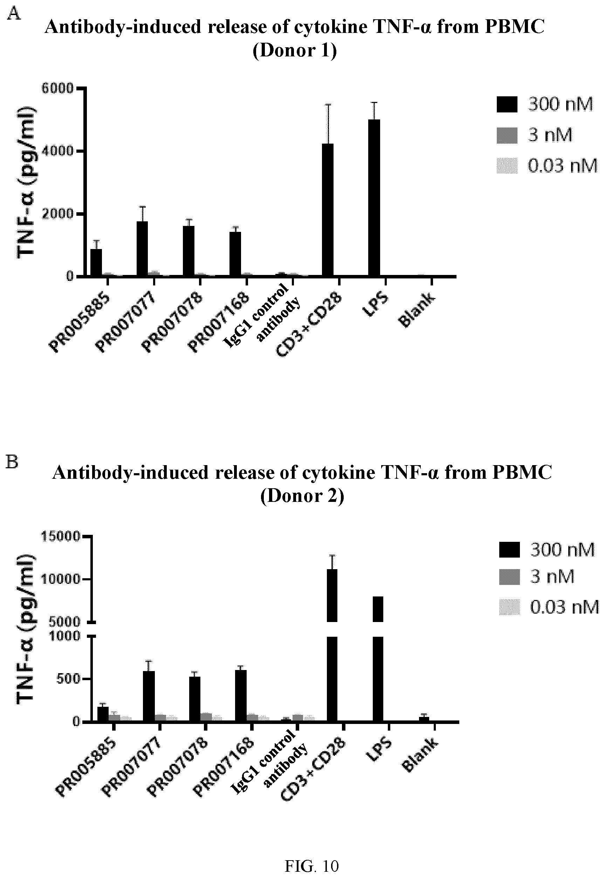

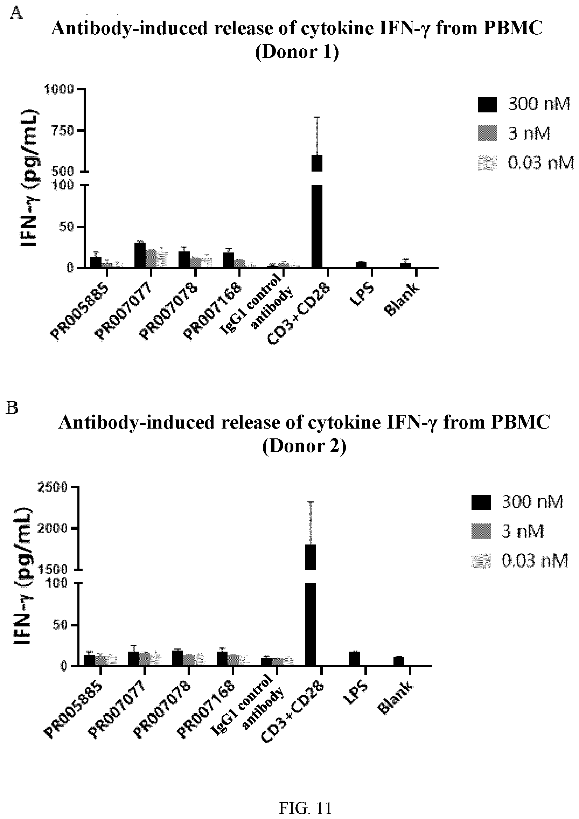

The results are shown in FIGs. 9A-9B, FIGs. 10A-10B, and FIGs. 11A-11B. The results show that the IL-6 and IFN-y expression induced by the bispecific antibody molecules PR007077, PR007078, and PR007168 was low, but a certain amount of TNF-α was expressed. The reactivity differs considerably in the case of different donors. PR005885 had the lowest cytokine expression compared with other molecules, suggesting that there might be a lower risk of cytokine storm.

### Example 9. In Vivo Drug Efficacy Experiment in NCG Mouse Tumor Model with Reconstruction of Human PBMC Immune System

In the OVCAR-3 models, on the day of cell inoculation, each NCG mouse was inoculated subcutaneously with 5 × 10⁶ OVCAR3 tumor cells, which were resuspended in a PBS/Matrigel (1:1) mixture (0.1 mL/mouse) for subcutaneous inoculation. When the mean tumor volume of the mice reached 110 mm³, 18 mice were divided into 3 groups, and each mouse was inoculated intravenously with 5 × 10⁶ human PBMCs, which were resuspended in 200 µL of PBS. The administration was started the next day with an administration cycle of once a week for a total of 3 times via intravenous administration. After the start of administration, the body weight and the tumor volume were measured twice a week. The tumor volume was calculated as follows: tumor volume (mm³) = 0.5 × long diameter of tumor × short diameter of tumor². The experimental observation ended on day 23 after administration and then all mice were euthanized.

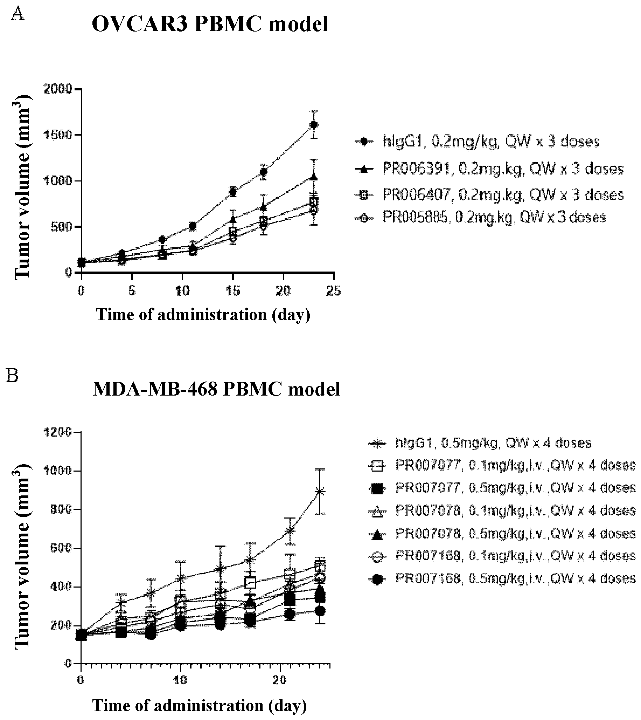

The results are shown in FIG. 12A. The mean tumor volume of the mice in the vehicle control group at day 23 after administration was 1612 mm³. The mean tumor volume of the test drug PR006391 (0.2 mg/kg) treatment group on day 23 after the administration was 1054 mm³, showing significant difference (p value was 0.04) from that of the vehicle control group, with the tumor growth inhibition rate TGI (%) being 34.6%. The mean tumor volume of the test drug PR006407 (0.2 mg/kg) treatment group on day 23 after the administration was 770 mm³, showing significant difference (p value was 0.001) from that of the vehicle control group, with the tumor growth inhibition rate TGI (%) being 52.2%. The mean tumor volume of the test drug PR005885 (0.2 mg/kg) treatment group on day 23 after the administration was 681 mm³, showing significant difference (p value was 0.001) from that of the vehicle control group, with the tumor growth inhibition rate TGI (%) being 57.77%.

In the MDA-MB-468 models, on the day of cell inoculation, each NCG mouse was inoculated subcutaneously with 5 × 10⁶ MDA-MB-468 tumor cells, which were resuspended in a PBS/Matrigel (1:1) mixture (0.1 mL/mouse) for subcutaneous inoculation. When the mean tumor volume of the mice reached 150 mm³, 42 mice were divided into 7 groups, and each mouse was inoculated intravenously with 5 × 10⁶ human PBMCs, which were resuspended in 200 µL of PBS. The administration was started the next day with an administration cycle of once a week for a total of 4 times via intravenous administration. After the start of administration, the body weight and the tumor volume were measured twice a week. The tumor volume was calculated as follows: tumor volume (mm³) = 0.5 × long diameter of tumor × short diameter of tumor². The experimental observation ended on day 23 after administration and then all mice were euthanized.

The results are shown in FIG. 12B. The mean tumor volume of the mice in the vehicle control group at day 23 after administration was 893 mm³. The mean tumor volume of the test drug PR007077 (0.1 mg/kg) treatment group on day 23 after the administration was 507 mm³, showing significant difference (p value was 0.01) from that of the vehicle control group, with the tumor growth inhibition rate TGI (%) being 43.16%. The mean tumor volume of the test drug PR007077 (0.5 mg/kg) treatment group on day 23 after the administration was 343 mm³, showing significant difference (p value was 0.0009) from that of the vehicle control group, with the tumor growth inhibition rate TGI (%) being 61.6%. The mean tumor volume of the test drug PR007078 (0.1 mg/kg) treatment group on day 23 after the administration was 464 mm³, showing significant difference (p value was 0.0067) from that of the vehicle control group, with the tumor growth inhibition rate TGI (%) being 47.9%. The mean tumor volume of the test drug PR007078 (0.5 mg/kg) treatment group on day 23 after the administration was 390 mm³, showing significant difference (p value was 0.0022) from that of the vehicle control group, with the tumor growth inhibition rate TGI (%) being 56.2%. The mean tumor volume of the test drug PR007168 (0.1 mg/kg) treatment group on day 23 after the administration was 446 mm³, showing significant difference (p value was 0.0092) from that of the vehicle control group, with the tumor growth inhibition rate TGI (%) being 50.1%. The mean tumor volume of the test drug PR007168 (0.5 mg/kg) treatment group on day 23 after the administration was 275 mm³, showing significant difference (p value was 0.0009) from that of the vehicle control group, with the tumor growth inhibition rate TGI (%) being 69.2%.

In the CT26-B7-H4 models, on the day of cell inoculation, each BALB/c-hCD3EDG knock in mouse was inoculated subcutaneously with 5 × 10⁵ CT26-B7-H4 tumor cells, which were resuspended in PBS (0.1 mL/mouse) for subcutaneous inoculation. When the mean tumor volume of the mice reached 84 mm³, 24 mice were divided into 4 groups and the administration started on the day of grouping, with an administration cycle of once a week for a total of 3 times via intravenous administration. After the start of administration, the body weight and the tumor volume were measured twice a week. The tumor volume was calculated as follows: tumor volume (mm³) = 0.5 × long diameter of tumor × short diameter of tumor². The experimental observation ended on day 20 after administration and then all mice were euthanized.

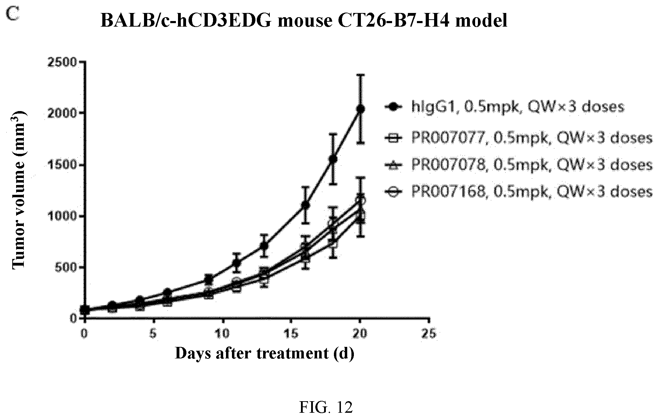

The results are shown in FIG. 12C. The mean tumor volume of the mice in the vehicle control group at day 20 after the administration was 2043 mm³. The mean tumor volume of the test drug PR007077 (0.5 mg/kg) treatment group on day 20 after the administration was 1007 mm³, showing significant difference (p value was 0.024) from that of the vehicle control group, with the tumor growth inhibition rate TGI (%) being 50.4%. The mean tumor volume of the test drug PR007078 (0.5 mg/kg) treatment group on day 20 after the administration was 1069 mm³, showing significant difference (p value was 0.031) from that of the vehicle control group, with the tumor growth inhibition rate TGI (%) being 45.5%. The mean tumor volume of the test drug PR007168 (0.5 mg/kg) treatment group on day 20 after the administration was 1155 mm³, showing significant difference (p value was 0.05) from that of the vehicle control group, with the tumor growth inhibition rate TGI (%) being 39.9%.

### Example 10. Pharmacokinetics in BALB/c Nude Mice

Six female BALB/c nude mice weighed 18-22 g were selected and subjected to drug administration via tail vein at a dose of 2.5 mg/kg; the whole blood of 3 mice in one group was collected prior to the administration and 5 min, 24 h (1 day), 4 days, and 10 days after the administration, and the whole blood of 3 mice in the other group was collected prior to the administration and 5 h, 2 days, 7 days, and 14 days after the administration. The whole blood was left to stand for 30 min for coagulation and centrifuged at 2000 rpm for 5 min at 4 °C, and the isolated serum sample was cryopreserved at -80 °C until it was taken for analysis. In this example, the drug concentration in the serum of mice was quantitatively determined by ELISA method. The ELISA Fc end overall detection method comprises capturing a fusion protein containing human Fc in the serum of mice using a goat anti-human Fc polyclonal antibody coating a 96-well plate and then adding a HRP-labeled goat anti-human Fc secondary antibody. ELISA B7-H4 end binding detection method comprises capturing B7-H4×CD3 bispecific antibody in the serum of mice using a recombinant human B7-H4 protein (Sino Biological, 10738-H08H) coating a 96-well plate and then adding a HRP-labeled goat anti-human Fc secondary antibody.

The plasma concentration data were analyzed using Phoenix WinNonlin software (version 8.2) by non-compartmental analysis (NCA) to evaluate the pharmacokinetics. The results are shown in Table 7 below.

The results show that the half-life of PR006407 in mice was about 6 days, and the half-life of PR007078 in mice was about 8 days, as calculated from the data of the first 14 days obtained under the Fc end overall detection method. The half-life of PR006407 in mice was only about 1 day, and the half-life of PR007078 in mice was about 12 days, as calculated from the data of the first 14 days obtained under the B7-H4 end binding detection method. This indicates that the disulfide bond-introduced antibody subjected to thermostability modification can improve the stability of the antibody in mice.

**Table 7. Pharmacokinetic parameters of PR006407 and corresponding disulfide bond-introduced antibody PR007078 subjected to thermostability modification**

| PK parameter | PR006407 | | PR007078 | |
|---|---|---|---|---|
| Assay method | Total antibody | B7-H4-binding antibody | Total antibody | B7-H4-binding antibody |
| CL (mL/hr/kg) | 2.15 | 4.2 | 1.5 | 2.27 |
| Vss (mL/kg) | 290 | 92.1 | 236 | 508 |
| Terminal t1/2 (hr) | 144 | 24.5 | 183 | 278 |
| AUClast (hr*µg/mL) | 1010 | 592 | 1393 | 846 |
| AUCINF (hr*µg/mL) | 1161 | 595 | 1665 | 1103 |
| MRTINF (hr) | 135 | 21.9 | 157 | 224 |
| C0 (µg/mL) | 41.8 | 43.8 | 37.4 | 31.4 |
| AUCINF_Free B7-H4/Total assay (%) | | 51.3 | | 66.2 |

## Claims

1. An anti-B7-H4 antibody, comprising a heavy chain variable region (VH); wherein
the VH comprises the following complementary determining regions (CDRs) or mutations thereof: a CDR1 having the amino acid sequence set forth in SEQ ID NO: 4 or 5, a CDR2 having the amino acid sequence set forth in SEQ ID NO: 15, and a CDR3 having the amino acid sequence set forth in SEQ ID NO: 24 or 25;
wherein the mutation is an insertion, a deletion, or a substitution of 3, 2, or 1 amino acid on the amino acid sequences of the CDRs.

2. The anti-B7-H4 antibody according to claim 1, wherein the mutation of the CDR2 is 2 or 1 amino acid substitution of G2P, S4G, and S5R/D/E/T on the amino acid sequence set forth in SEQ ID NO: 15; the CDR2 has the amino acid sequence preferably set forth in any one of SEQ ID NOs: 11-14 and SEQ ID NOs: 16-18.

3. The anti-B7-H4 antibody according to claim 1 or 2, wherein the CDR1, the CDR2, and the CDR3 comprised in the VH have amino acid sequences set forth in SEQ ID NOs: 4, 11, and 24, respectively; or
the CDR1, the CDR2, and the CDR3 comprised in the VH have amino acid sequences set forth in SEQ ID NOs: 5, 12, and 25, respectively; or
the CDR1, the CDR2, and the CDR3 comprised in the VH have amino acid sequences set forth in SEQ ID NOs: 4, 17, and 24, respectively; or
the CDR1, the CDR2, and the CDR3 comprised in the VH have amino acid sequences set forth in SEQ ID NOs: 5, 18, and 25, respectively; or
the CDR1, the CDR2, and the CDR3 comprised in the VH have amino acid sequences set forth in SEQ ID NOs: 4, 13, and 24, respectively; or
the CDR1, the CDR2, and the CDR3 comprised in the VH have amino acid sequences set forth in SEQ ID NOs: 4, 14, and 24, respectively; or
the CDR1, the CDR2, and the CDR3 comprised in the VH have amino acid sequences set forth in SEQ ID NOs: 4, 15, and 24, respectively; or
the CDR1, the CDR2, and the CDR3 comprised in the VH have amino acid sequences set forth in SEQ ID NOs: 4, 16, and 24, respectively.

4. The anti-B7-H4 antibody according to any one of claims 1-3, wherein framework regions of the VH are framework regions of a human VH;
preferably, the framework regions of the human VH comprise a FWR1 having the amino acid sequence set forth in SEQ ID NO: 2, a FWR2 having the amino acid sequence set forth in any one of SEQ ID NOs: 7-9, a FWR3 having the amino acid sequence set forth in any one of SEQ ID NOs: 20-22, and a FWR4 having the amino acid sequence set forth in SEQ ID NO: 26 or 27;
more preferably, the VH comprises the amino acid sequence set forth in any one of SEQ ID NOs: 36-45.

5. The anti-B7-H4 antibody according to any one of claims 1-4, being a full-length antibody, a Fab, a Fab', a F(ab')₂, a Fv, preferably a scFv, a bispecific antibody, a multispecific antibody, a heavy-chain antibody, or a single-domain antibody, wherein preferably, when the anti-B7-H4 antibody is a heavy-chain antibody, the heavy-chain antibody comprises the amino acid sequence set forth in any one of SEQ ID NOs: 48-57.

6. A bispecific antibody, comprising a protein functional region A and a protein functional region B, wherein the protein functional region B targets B7-H4, the protein functional region A targets non-B7-H4, and the bispecific antibody is selected from the following a) or b):
a) the protein functional region B is selected from the anti-B7-H4 antibody according to any one of claims 1-5;
preferably, the structure of the bispecific antibody comprises a polypeptide chain 1, a polypeptide chain 2, and a polypeptide chain 3, wherein the polypeptide chain 1 is shown as the formula N'-VL_A-CL-C', the polypeptide chain 2 is shown as the formula N'-VH_A-CH1-hinge region-CH2-CH3-C', and the polypeptide chain 3 is shown as the formula N'-VH_B1-linker-VH_B2-hinge region-CH2-CH3-C' or the formula N'-VH_B-hinge region-CH2-CH3-C', wherein the VL_A and the VH_A are a VL and a VH of the protein functional region A respectively, the VH_B1 and the VH_B2 are VHs of the protein functional region B, and the VH_B1 and the VH_B2 can be the same or different;
b) the protein functional region B comprises a HCDR1 set forth in SEQ ID NO: 72, a HCDR2 set forth in SEQ ID NO. 74, a HCDR3 set forth in SEQ ID NO: 76, a LCDR1 set forth in SEQ ID NO: 78, a LCDR2 set forth in SEQ ID NO: 80, and a LCDR3 set forth in SEQ ID NO: 82;
preferably, the structure of the bispecific antibody comprises a polypeptide chain 1, a polypeptide chain 2, and a polypeptide chain 3, wherein the polypeptide chain 1 is shown as the formula N'-VL_A-CL-C', the polypeptide chain 2 is shown as the formula N'-VH_A-CH1-hinge region-CH2-CH3-C', and the polypeptide chain 3 is shown as the formula N'-VL_B-linker-VH _B-hinge region-CH2-CH3-C', wherein, the VL_A and the VH_A are a VL and a VH of the protein functional region A respectively, and the VL_B and the VH_B are a VL and a VH of the protein functional region B.

7. The bispecific antibody according to claim 6, wherein the protein functional region A is an anti-CD3 antibody comprising a heavy chain variable region (VH) and a light chain variable region (VL), wherein the VH comprises a VH CDR1 having the amino acid sequence set forth in SEQ ID NO: 3, a VH CDR2 having the amino acid sequence set forth in SEQ ID NO: 10, and a VH CDR3 having the amino acid sequence set forth in SEQ ID NO: 23, and the VL comprises a VL CDR1 having the amino acid sequence set forth in SEQ ID NO: 29, a VL CDR2 having the amino acid sequence set forth in SEQ ID NO: 31, and a VL CDR3 having the amino acid sequence set forth in SEQ ID NO: 33;
preferably, the anti-CD3 antibody comprises a VH having the amino acid sequence set forth in SEQ ID NO: 35 and a VL having the amino acid sequence set forth in SEQ ID NO: 46;
more preferably, the anti-CD3 antibody comprises a heavy chain having the amino acid sequence set forth in SEQ ID NO: 47 and a light chain having the amino acid sequence set forth in SEQ ID NO: 58.

8. The bispecific antibody according to claim 6 or 7, wherein the bispecific antibody comprises:
a polypeptide chain 1 having the amino acid sequence set forth in SEQ ID NO: 58, a polypeptide chain 2 having the amino acid sequence set forth in SEQ ID NO: 59, and a polypeptide chain 3 having the amino acid sequence set forth in SEQ ID NO: 60; or
a polypeptide chain 1 having the amino acid sequence set forth in SEQ ID NO: 58, a polypeptide chain 2 having the amino acid sequence set forth in SEQ ID NO: 59, and a polypeptide chain 3 having the amino acid sequence set forth in SEQ ID NO: 61; or
a polypeptide chain 1 having the amino acid sequence set forth in SEQ ID NO: 58, a polypeptide chain 2 having the amino acid sequence set forth in SEQ ID NO: 59, and a polypeptide chain 3 having the amino acid sequence set forth in SEQ ID NO: 62; or
a polypeptide chain 1 having the amino acid sequence set forth in SEQ ID NO: 58, a polypeptide chain 2 having the amino acid sequence set forth in SEQ ID NO: 59, and a polypeptide chain 3 having the amino acid sequence set forth in SEQ ID NO: 63; or
a polypeptide chain 1 having the amino acid sequence set forth in SEQ ID NO: 58, a polypeptide chain 2 having the amino acid sequence set forth in SEQ ID NO: 59, and a polypeptide chain 3 having the amino acid sequence set forth in SEQ ID NO: 64; or
a polypeptide chain 1 having the amino acid sequence set forth in SEQ ID NO: 58, a polypeptide chain 2 having the amino acid sequence set forth in SEQ ID NO: 59, and a polypeptide chain 3 having the amino acid sequence set forth in SEQ ID NO: 65; or
a polypeptide chain 1 having the amino acid sequence set forth in SEQ ID NO: 58, a polypeptide chain 2 having the amino acid sequence set forth in SEQ ID NO: 59, and a polypeptide chain 3 having the amino acid sequence set forth in SEQ ID NO: 69; or
a polypeptide chain 1 having the amino acid sequence set forth in SEQ ID NO: 58, a polypeptide chain 2 having the amino acid sequence set forth in SEQ ID NO: 59, and a polypeptide chain 3 having the amino acid sequence set forth in SEQ ID NO: 70; or
the bispecific antibody comprises a polypeptide chain 1 having the amino acid sequence set forth in SEQ ID NO: 58, a polypeptide chain 2 having the amino acid sequence set forth in SEQ ID NO: 59, and a polypeptide chain 3 having the amino acid sequence set forth in SEQ ID NO: 86.

9. An isolated nucleic acid, encoding the anti-B7-H4 antibody according to any one of claims 1-5 or the bispecific antibody according to any one of claims 6-8.

10. A recombinant expression vector, comprising the isolated nucleic acid according to claim 9, wherein
preferably, the expression vector comprises a eukaryotic cell expression vector and/or a prokaryotic cell expression vector.

11. A transformant, comprising the recombinant expression vector according to claim 10, wherein
preferably, the host cell of the transformant is a prokaryotic cell and/or a eukaryotic cell, wherein the prokaryotic cell is preferably an *E*. *coli* cell such as a TG1 or BL21 cell, and the eukaryotic cell is preferably a HEK293 cell or a CHO cell.

12. A chimeric antigen receptor, comprising the anti-B7-H4 antibody according to any one of claims 1-5 or the bispecific antibody according to any one of claims 6-8.

13. A genetically modified cell, comprising the chimeric antigen receptor according to claim 12, wherein
preferably, the genetically modified cell is a eukaryotic cell, preferably an isolated human cell, and more preferably an immune cell such as a T cell or an NK cell.

14. A method for preparing an anti-B7-H4 antibody or a bispecific antibody, comprising the following steps: culturing the transformant according to claim 11 and obtaining the anti-B7-H4 antibody or the bispecific antibody from the culture.

15. An antibody-drug conjugate, comprising an antibody moiety and a conjugate moiety, wherein the antibody moiety comprises the anti-B7-H4 antibody according to any one of claims 1-5 or the bispecific antibody according to any one of claims 6-8, and the conjugate moiety comprises a detectable label, a drug, a toxin, a cytokine, a radionuclide, an enzyme, or a combination thereof, the antibody moiety and the conjugate moiety being conjugated via a chemical bond or a linker.

16. A pharmaceutical composition, comprising the anti-B7-H4 antibody according to any one of claims 1-5, the bispecific antibody according to any one of claims 6-8, the chimeric antigen receptor according to claim 12, the genetically modified cell according to claim 13, and/or the antibody-drug conjugate according to claim 15, wherein
preferably, the pharmaceutical composition is in a liquid dosage form, a gas dosage form, a solid dosage form, and a semi-solid dosage form, and/or the pharmaceutical composition can be administered orally, by injection, nasally, transdermally, or transmucosally;
further preferably, the pharmaceutical composition further comprises a combination therapeutic agent comprising a chemotherapeutic agent, a radiotherapeutic agent, an immunosuppressant, and/or a cytotoxic drug.

17. Use of the anti-B7-H4 antibody according to any one of claims 1-5, the bispecific antibody according to any one of claims 6-8, the chimeric antigen receptor according to claim 12, the genetically modified cell according to claim 13, the antibody-drug conjugate according to claim 15, and/or the pharmaceutical composition according to claim 16 in the preparation of a medicament, a kit, and/or an administration device for treating and/or preventing cancer, wherein
preferably, the cancer is selected from the group consisting of lung cancer, breast cancer such as triple negative breast cancer, ovarian cancer, endometrioma, stomach cancer, small intestine cancer, colon cancer, rectal cancer, pancreatic cancer, kidney cancer, bladder cancer, prostate cancer, bile duct cancer, esophageal cancer, and osteosarcoma.

18. A kit, comprising the antibody according to any one of claims 1-5, the bispecific antibody according to any one of claims 6-8, the chimeric antigen receptor according to claim 12, the genetically modified cell according to claim 13, the antibody-drug conjugate according to claim 15, and/or the pharmaceutical composition according to claim 16, and optionally, instructions.

19. An administration device, comprising: (1) an infusion module for administering to a subject in need thereof the pharmaceutical composition according to claim 16, and (2) optionally a pharmacodynamic monitoring module.

20. A method for detecting B7-H4, comprising detecting using the anti-B7-H4 antibody according to any one of claims 1-5 and/or the bispecific antibody according to any one of claims 6-8, wherein preferably, the method is for non-diagnostic and/or non-therapeutic purposes.
